# EUROPÄISCHE PATENTSCHRIFT

(11) **EP 2 175 024 B1**
(45) Veröffentlichungstag und Bekanntmachung des Hinweises auf die Patenterteilung: **04.12.2013**
(21) Anmeldenummer: 09174021.7
(22) Anmeldetag: 09.10.2006
(51) Int. Cl.: C12P 7/40, C12P 7/42, C12P 7/44

(54) **Mikrobiologische Herstellung von 3-Hydroxypropionsäure**
Microbiological production of 3-hydroxypropionic acid
Fabrication microbiologique d'acides 3-hydroxypropion

(30) Priorität: 10.10.2005 DE 102005048818
(43) Veröffentlichungstag der Anmeldung: 14.04.2010
(62) Teilanmeldung aus: 06807074.7
(73) Patentinhaber: Evonik Degussa GmbH, 45128 Essen (DE); Forschungszentrum Jülich Gmbh, 52428 Jülich (DE)
(72) Erfinder: Marx, Achim Dr., 63571 Gelnhausen (DE); Wendisch, Volker F. Prof. Dr., 52428 Jülich (DE); Rittmann, Doris, 52428 Jülich (DE); Buchholz, Stefan Dr., 63456 Hanau (DE)

(56) Entgegenhaltungen:
- EP-A1- 1 083 225
- WO-A2-03/062173
- DE-A1- 19 831 609
- US-A1- 2005 221 466
- ADRIE J J STRAATHOF ET AL: "Feasibility of acrylic acid production by fermentation" APPLIED MICROBIOLOGY AND BIOTECHNOLOGY, SPRINGER, BERLIN, DE, Bd. 67, Nr. 6, 1. Juni 2005 (2005-06-01), Seiten 727-734, XP019331875 ISSN: 1432-0614
- GEORGI ET AL: "Lysine and glutamate production by Corynebacterium glutamicum on glucose, fructose and sucrose: Roles of malic enzyme and fructose-1,6-bisphosphatase" METABOLIC ENGINEERING, ACADEMIC PRESS,, US, Bd. 7, Nr. 4, Juli 2005 (2005-07), Seiten 291-301, XP005052640 ISSN: 1096-7176
- XINGLIN JIANG ET AL: "Biosynthetic pathways for 3-hydroxypropionic acid production" APPLIED MICROBIOLOGY AND BIOTECHNOLOGY, SPRINGER, BERLIN, DE, Bd. 82, Nr. 6, 17. Februar 2009 (2009-02-17), Seiten 995-1003, XP019705542 ISSN: 1432-0614
- ANDERSON CATRIONA M H ET AL: "Indirect regulation of the intestinal H+-coupled amino acid transporter hPAT1 (SLC36A1)", JOURNAL OF CELLULAR PHYSIOLOGY, vol. 204, no. 2, August 2005 (2005-08), pages 604-613, ISSN: 0021-9541
- BRECHTEL CASEY E ET AL: "4-Aminobutyrate (GABA) transporters from the amine-polyamine-choline superfamily: Substrate specificity and ligand recognition profile of the 4-aminobutyrate permease from Bacillus subtilis", BIOCHEMICAL JOURNAL, vol. 333, no. 3, 1 August 1998 (1998-08-01), pages 565-571, ISSN: 0264-6021
- SCHNEIDER FRANK ET AL: "Identification and characterization of the main beta-alanine uptake system in Escherichia coli", APPLIED MICROBIOLOGY AND BIOTECHNOLOGY, vol. 65, no. 5, October 2004 (2004-10), pages 576-582, ISSN: 0175-7598
- GUIMBAL CECILE ET AL: "Phylogenetic conservation of 4-aminobutyric acid (GABA) transporter isoforms: Cloning and pharmacological characterization of a GABA/beta-alanine transporter from Torpedo", EUROPEAN JOURNAL OF BIOCHEMISTRY, vol. 234, no. 3, 1995, pages 794-800, ISSN: 0014-2956
- MUNCK LARS KRISTIAN ET AL: "Transport of glycine and lysine on the chloride-dependent beta-alanine (B-0,+) carrier in rabbit small intestine", BIOCHIMICA ET BIOPHYSICA ACTA, vol. 1235, no. 1, 1995, pages 93-99, ISSN: 0006-3002
- SHUTTLEWORTH T J ET AL: "BETA ALANINE TRANSPORT IN THE ISOLATED HEPATOCYTES OF THE ELASMOBRANCH RAJA-ERINACEA/", JOURNAL OF EXPERIMENTAL ZOOLOGY, vol. 231, no. 1, 1984, pages 39-44, ISSN: 0022-104X
- B. Winnen et al.: "Genomic Analyses of Transporter Proteins in Corynebacterium glutamicum and Corynebacterium efficiens, Chapter 8" In: Lothar Eggeling et al.: "Handbook of Corynebacterium glutamicum", 2005, Taylor & Francis pages 149-186,

### SEQUENCE LISTING

<110> Degussa AG
<120> Mikrobiologische Herstellung von 3-Hydroxypropionsäure
<130> 050187 OC
<160> 7
<170> PatentIn version 3.1
<210> 1
   <211> 11959
   <212> DNA
   <213> Artificial
<220>
   <223> Plasmid pVWEx1-glpKD-panD
<400> 1
   aagcttgcat gcctgcaggt cgacaaggag atatagatga ctgaaaaaaa atatatcgtt   60
   gcgctcgacc agggcaccac cagctcccgc gcggtcgtaa tggatcacga tgccaatatc  120
   attagcgtgt cgcagcgcga atttgagcaa atctacccaa aaccaggttg ggtagaacac  180
   gacccaatgg aaatctgggc cacccaaagc tccacgctgg tagaagtgct ggcgaaagcc  240
   gatatcagtt ccgatcaaat tgcagctatc ggtattacga accagcgtga aaccactatt  300
   gtctgggaaa aagaaaccgg caagcctatc tataacgcca ttgtctggca gtgccgtcgt  360
   accgcagaaa tctgcgagca tttaaaacgt gacggtttag aagattatat ccgcagcaat  420
   accggtctgg tgattgaccc gtacttttct ggcaccaaag tgaagtggat cctcgaccat  480
   gtggaaggct ctcgcgagcg tgcacgtcgt ggtgaattgc tgtttggtac ggttgatacg  540
   tggcttatct ggaaaatgac tcagggccgt gtccatgtga ccgattacac caacgcctct  600
   cgtaccatgt tgttcaacat ccataccctg gactgggacg acaaaatgct ggaagtgctg  660
   gatattccgc gcgagatgct gccagaagtg cgtcgttctt ccgaagtata cggtcagact  720
   aacattggcg gcaaaggcgg cacgcgtatt ccaatctccg ggatcgccgg tgaccagcag  780
   gccgcgctgt ttggtcagtt gtgcgtgaaa gaagggatgg cgaagaacac ctatggcact  840
   ggctgcttta tgctgatgaa cactggcgag aaagcggtga aatcagaaaa cggcctgctg  900
   accaccatcg cctgcggccc gactggcgaa gtgaactatg cgttggaagg tgcggtgttt  960
   atggcaggcg catccattca gtggctgcgc gatgaaatga agttgattaa cgacgcctac 1020
   gattccgaat atttcgccac caaagtgcaa aacaccaatg gtgtgtatgt ggttccggca 1080
   tttaccgggc tgggtgcgcc gtactgggac ccgtatgcgc gcggggcgat tttcggtctg 1140
   actcgtgggg tgaacgctaa ccacattata cgcgcgacgc tggagtctat tgcttatcag 1200
   acgcgtgacg tgctggaagc gatgcaggcc gactctggta tccgtctgca cgccctgcgc 1260
   gtggatggtg gcgcagtagc aaacaatttc ctgatgcagt tccagtccga tattctcggc 1320
   acccgcgttg agcgcccgga agtgcgcgaa gtcaccgcat tgggtgcggc ctatctcgca 1380
   ggcctggcgg ttggcttctg gcagaacctc gacgagctgc aagagaaagc ggtgattgag 1440
   cgcgagttcc gtccaggcat cgaaaccact gagcgtaatt accgttacgc aggctggaaa 1500
   aaagcggtta aacgcgcgat ggcgtgggaa gaacacgacg aataaatcac tagaaaggag 1560
   atatagatgg aaaccaaaga tctgattgtg atagggggcg gcatcaatgg tgctggtatc 1620
   gcggcagacg ccgctggacg cggtttatcc gtgctgatgc tggaggcgca ggatctcgct 1680
   tgcgcgacct cttccgccag ttcaaaactc attcacggtg gcctgcgcta ccttgagcac 1740
   tatgaattcc gcctggtcag cgaggcgctg gctgaacgtg aagtgctgct gaaaatggcc 1800
   ccgcatatcg ccttcccgat gcgttttcgc ctgccacatc gtccgcatct gcgcccggcg 1860
   tggatgattc gcattggtct gtttatgtac gatcatctgg gtaaacgcac cagcttgccg 1920
   ggatcaactg gtttgcgttt tggcgcaaat tcagtgttaa aaccggaaat taagcgcgga 1980
   ttcgaatatt ctgactgttg ggtagacgac gcccgtctgg tactcgccaa cgcccagatg 2040
   gtggtgcgta aaggcggcga agtgcttact cggactcgcg ccacctctgc tcgccgcgaa 2100
   aacggcctgt ggattgtgga agcggaagat atcgataccg gcaaaaaata tagctggcaa 2160
   gcgcgcggct tggttaacgc caccggcccg tgggtgaaac agttcttcga cgacgggatg 2220
   catctgcctt cgccttatgg cattcgcctg atcaaaggca gccatattgt ggtgccgcgc 2280
   gtgcataccc agaagcaagc ctacattctg caaaacgaag ataaacgtat tgtgttcgtg 2340
   atcccgtgga tggacgagtt ttccatcatc ggcactaccg atgtcgagta caaaggcgat 2400
   ccgaaagcgg tgaagattga agagagtgaa atcaattacc tgctgaatgt gtataacacg 2460
   cactttaaaa agcagttaag ccgtgacgat atcgtctgga cctactccgg tgtgcgtccg 2520
   ctgtgtgatg atgagtccga ctcgccgcag gctattaccc gtgattacac ccttgatatt 2580
   catgatgaaa atggcaaagc accgctgctg tcggtattcg gcggtaagct gaccacctac 2640
   cgaaaactgg cggaacatgc gctggaaaaa ctaacgccgt attatcaggg tattggcccg 2700
   gcatggacga aagagagtgt gctaccgggt ggcgccattg aaggcgaccg cgacgattat 2760
   gccgctcgcc tgcgccgccg ctatccgttc ctgactgaat cgctggcgcg tcattacgct 2820
   cgcacttacg gcagcaacag cgagctgctg ctcggcaatg cgggaacggt aagcgatctc 2880
   ggggaagatt tcggtcatga gttctacgaa gcggagctga aatacctggt ggatcacgaa 2940
   tgggtccgcc gcgccgacga cgccctgtgg cgtcgcacaa aacaaggcat gtggctaaat 3000
   gcggatcaac aatctcgtgt gagtcagtgg ctggtggagt atacgcagca gaggttatcg 3060
   ctggcgtcgt aatctagtgc ggccgcctgc aggtcgactc tagtaaggag atatagatgc 3120
   tgcgcaccat cctcggaagt aagattcacc gagccactgt cactcaagct gatctagatt 3180
   atgttggctc tgtaaccatc gacgccgacc tggttcacgc cgccggattg atcgaaggcg 3240
   aaaaagttgc catcgtagac atcaccaacg gcgctcgtct ggaaacttat gtcattgtgg 3300
   gcgacgccgg aacgggcaat atttgcatca atggtgccgc tgcacacctt attaatcctg 3360
   gcgatcttgt gatcatcatg agctaccttc aggcaactga tgcggaagcc aaggcgtatg 3420
   agccaaagat tgtgcacgtg gacgccgaca accgcatcgt tgcgctcggc aacgatcttg 3480
   cggaagcact acctggatcc gggcttttga cgtcgagaag catttagggt accgagctcg 3540
   aattcactgg ccgtcgtttt acagccaagc ttggctgttt tggcggatga gagaagattt 3600
   tcagcctgat acagattaaa tcagaacgca gaagcggtct gataaaacag aatttgcctg 3660
   gcggcagtag cgcggtggtc ccacctgacc ccatgccgaa ctcagaagtg aaacgccgta 3720
   gcgccgatgg tagtgtgggg tctccccatg cgagagtagg gaactgccag gcatcaaata 3780
   aaacgaaagg ctcagtcgaa agactgggcc tttcgtttta tctgttgttt gtcggtgaac 3840
   gctctcctga gtaggacaaa tccgccggga gcggatttga acgttgcgaa gcaacggccc 3900
   ggagggtggc gggcaggacg cccgccataa actgccaggc atcaaattaa gcagaaggcc 3960
   atcctgacgg atggcctttt tgcgtttcta caaactcttt tgtttatttt tctaaataca 4020
   ttcaaatatg tatccgctca tgagacaata accctgataa atgcttcaat aatattgaaa 4080
   aaggaagagt atgagtattc aacatttccg tgtcgccctt attccctttt ttgcggcatt 4140
   ttgccttcct gtttttgctc acccagaaac gctggtgaaa gtaaaagatg ctgaagatca 4200
   gttgggtgca cgagtgggtt acatcgaact ggatctcaac agcggtaaga tccttgagag 4260
   ttttcgcccc gaagaacgtt ttccaatgat gagcactttt gatccccctg cggcgtcgct 4320
   gatcgccctc gcgacgttgt gcgggtggct tgtccctgag ggcgctgcga cagatagcta 4380
   aaaatctgcg tcaggatcgc cgtagagcgc gcgtcgcgtc gattggaggc ttcccctttg 4440
   gttgacggtc ttcaatcgct ctacggcgat cctgacgctt ttttgttgcg taccgtcgat 4500
   cgttttattt ctgtcgatcc cgaaaaagtt tttgcctttt gtaaaaaact tctcggtcgc 4560
   cccgcaaatt ttcgattcca gattttttaa aaaccaagcc agaaatacga cacaccgttt 4620
   gcagataatc tgtctttcgg aaaaatcaag tgcgatacaa aatttttagc acccctgagc 4680
   tgcgcaaagt cccgcttcgt gaaaattttc gtgccgcgtg attttccgcc aaaaacttta 4740
   acgaacgttc gttataatgg tgtcatgacc ttcacgacga agtaccaaaa ttggcccgaa 4800
   tcatcagcta tggatctctc tgatgtcgcg ctggagtccg acgcgctcga tgctgccgtc 4860
   gatttaaaaa cggtgatcgg atttttccga gctctcgata cgacggacgc gccagcatca 4920
   cgagactggg ccagtgccgc gagcgaccta gaaactctcg tggcggatct tgaggagctg 4980
   gctgacgagc tgcgtgctcg gcagcgccag gaggacgcac agtagtggag gatcgaatca 5040
   gttgcgccta ctgcggtggc ctgattcctc cccggcctga cccgcgagga cggcgcgcaa 5100
   aatattgctc agatgcgtgt cgtgccgcag ccagccgcga gcgcgccaac aaacgccacg 5160
   ccgaggagct ggaggcggct aggtcgcaaa tggcgctgga agtgcgtccc ccgagcgaaa 5220
   ttttggccat ggtcgtcaca gagctggaag cggcagcgag aattatccgc gatcgtggcg 5280
   cggtgcccgc aggcatgaca aacatcgtaa atgccgcgtt tcgtgtggcc gtggccgccc 5340
   aggacgtgtc agcgccgcca ccacctgcac cgaatcggca gcagcgtcgc gcgtcgaaaa 5400
   agcgcacagg cggcaagaag cgataagctg cacgaatacc tgaaaaatgt tgaacgcccc 5460
   gtgagcggta actcacaggg cgtcggctaa cccccagtcc aaacctggga gaaagcgctc 5520
   aaaaatgact ctagcggatt cacgagacat tgacacaccg gcctggaaat tttccgctga 5580
   tctgttcgac acccatcccg agctcgcgct gcgatcacgt ggctggacga gcgaagaccg 5640
   ccgcgaattc ctcgctcacc tgggcagaga aaatttccag ggcagcaaga cccgcgactt 5700
   cgccagcgct tggatcaaag acccggacac gggagaaaca cagccgaagt tataccgagt 5760
   tggttcaaaa tcgcttgccc ggtgccagta tgttgctctg acgcacgcgc agcacgcagc 5820
   cgtgcttgtc ctggacattg atgtgccgag ccaccaggcc ggcgggaaaa tcgagcacgt 5880
   aaaccccgag gtctacgcga ttttggagcg ctgggcacgc ctggaaaaag cgccagcttg 5940
   gatcggcgtg aatccactga gcgggaaatg ccagctcatc tggctcattg atccggtgta 6000
   tgccgcagca ggcatgagca gcccgaatat gcgcctgctg gctgcaacga ccgaggaaat 6060
   gacccgcgtt ttcggcgctg accaggcttt ttcacatagg ctgagccggt ggccactgca 6120
   cgtctccgac gatcccaccg cgtaccgctg gcatgcccag cacaatcgcg tggatcgcct 6180
   agctgatctt atggaggttg ctcgcatgat ctcaggcaca gaaaaaccta aaaaacgcta 6240
   tgagcaggag ttttctagcg gacgggcacg tatcgaagcg gcaagaaaag ccactgcgga 6300
   agcaaaagca cttgccacgc ttgaagcaag cctgccgagc gccgctgaag cgtctggaga 6360
   gctgatcgac ggcgtccgtg tcctctggac tgctccaggg cgtgccgccc gtgatgagac 6420
   ggcttttcgc cacgctttga ctgtgggata ccagttaaaa gcggctggtg agcgcctaaa 6480
   agacaccaag atcatcgacg cctacgagcg tgcctacacc gtcgctcagg cggtcggagc 6540
   agacggccgt gagcctgatc tgccgccgat gcgtgaccgc cagacgatgg cgcgacgtgt 6600
   gcgcggctac gtcgctaaag gccagccagt cgtccctgct cgtcagacag agacgcagag 6660
   cagccgaggg cgaaaagctc tggccactat gggaagacgt ggcggtaaaa aggccgcaga 6720
   acgctggaaa gacccaaaca gtgagtacgc ccgagcacag cgagaaaaac tagctaagtc 6780
   cagtcaacga caagctagga aagctaaagg aaatcgcttg accattgcag gttggtttat 6840
   gactgttgag ggagagactg gctcgtggcc gacaatcaat gaagctatgt ctgaatttag 6900
   cgtgtcacgt cagaccgtga atagagcact taagtctgcg ggcattgaac ttccacgagg 6960
   acgccgtaaa gcttcccagt aaatgtgcca tctcgtaggc agaaaacggt tccccccgta 7020
   ggggtctctc tcttggcctc ctttctaggt cgggctgatt gctcttgaag ctctctaggg 7080
   gggctcacac cataggcaga taacggttcc ccaccggctc acctcgtaag cgcacaagga 7140
   ctgctcccaa agatcttcaa agccactgcc gcgactccgc ttcgcgaagc cttgccccgc 7200
   ggaaatttcc tccaccgagt tcgtgcacac ccctatgcca agcttctttc accctaaatt 7260
   cgagagattg gattcttacc gtggaaattc ttcgcaaaaa tcgtcccctg atcgcccttg 7320
   cgacgttgct cgcggcggtg ccgctggttg cgcttggctt gaccgacttg atcctccggc 7380
   gttcagcctg tgccacagcc gacaggatgg tgaccaccat ttgccccata tcaccgtcgg 7440
   tactgatccc gtcgtcaata aaccgaaccg ctacaccctg agcatcaaac tcttttatca 7500
   gttggatcat gtcggcggtg tcgcggccaa gacggtcgag cttcttcacc agaatgacat 7560
   caccttcctc caccttcatc ctcagcaaat ccagcccttc ccgatctgtt gaactgccgg 7620
   atgccttgtc ggtaaagatg cggttagctt ttacccctgc atctttgagc gctgaggtct 7680
   gcctcgtgaa gaaggtgttg ctgactcata ccaggcctga atcgccccat catccagcca 7740
   gaaagtgagg gagccacggt tgatgagagc tttgttgtag gtggaccagt tggtgatttt 7800
   gaacttttgc tttgccacgg aacggtctgc gttgtcggga agatgcgtga tctgatcctt 7860
   caactcagca aaagttcgat ttattcaaca aagccgccgt cccgtcaagt cagcgtaatg 7920
   ctctgccagt gttacaacca attaaccaat tctgattaga aaaactcatc gagcatcaaa 7980
   tgaaactgca atttattcat atcaggatta tcaataccat atttttgaaa aagccgtttc 8040
   tgtaatgaag gagaaaactc accgaggcag ttccatagga tggcaagatc ctggtatcgg 8100
   tctgcgattc cgactcgtcc aacatcaata caacctatta atttcccctc gtcaaaaata 8160
   aggttatcaa gtgagaaatc accatgagtg acgactgaat ccggtgagaa tggcaaaagc 8220
   ttatgcattt ctttccagac ttgttcaaca ggccagccat tacgctcgtc atcaaaatca 8280
   ctcgcatcaa ccaaaccgtt attcattcgt gattgcgcct gagcgagacg aaatacgcga 8340
   tcgctgttaa aaggacaatt acaaacagga atcgaatgca accggcgcag gaacactgcc 8400
   agcgcatcaa caatattttc acctgaatca ggatattctt ctaatacctg gaatgctgtt 8460
   ttcccgggga tcgcagtggt gagtaaccat gcatcatcag gagtacggat aaaatgcttg 8520
   atggtcggaa gaggcataaa ttccgtcagc cagtttagtc tgaccatctc atctgtaaca 8580
   tcattggcaa cgctaccttt gccatgtttc agaaacaact ctggcgcatc gggcttccca 8640
   tacaatcgat agattgtcgc acctgattgc ccgacattat cgcgagccca tttataccca 8700
   tataaatcag catccatgtt ggaatttaat cgcggcctcg agcaagacgt ttcccgttga 8760
   atatggctca taacacccct tgtattactg tttatgtaag cagacagttt tattgttcat 8820
   gatgatatat ttttatcttg tgcaatgtaa catcagagat tttgagacac aacgtggctt 8880
   tgttgaataa atcgaacttt tgctgagttg aaggatcaga tcacgcatct tcccgacaac 8940
   gcagaccgtt ccgtggcaaa gcaaaagttc aaaatcacca actggtccac ctacaacaaa 9000
   gctctcatca accgtggctc cctcactttc tggctggatg atggggcgat tcaggcctgg 9060
   tatgagtcag caacaccttc ttcacgaggc agacctcagc gctagcggag tgtatactgg 9120
   cttactatgt tggcactgat gagggtgtca gtgaagtgct tcatgtggca ggagaaaaaa 9180
   ggctgcaccg gtgcgtcagc agaatatgtg atacaggata tattccgctt cctcgctcac 9240
   tgactcgcta cgctcggtcg ttcgactgcg gcgagcggaa atggcttacg aacggggcgg 9300
   agatttcctg gaagatgcca ggaagatact taacagggaa gtgagagggc cgcggcaaag 9360
   ccgtttttcc ataggctccg cccccctgac aagcatcacg aaatctgacg ctcaaatcag 9420
   tggtggcgaa acccgacagg actataaaga taccaggcgt ttccccctgg cggctccctc 9480
   gtgcgctctc ctgttcctgc ctttcggttt accggtgtca ttccgctgtt atggccgcgt 9540
   ttgtctcatt ccacgcctga cactcagttc cgggtaggca gttcgctcca agctggactg 9600
   tatgcacgaa ccccccgttc agtccgaccg ctgcgcctta tccggtaact atcgtcttga 9660
   gtccaacccg gaaagacatg caaaagcacc actggcagca gccactggta attgatttag 9720
   aggagttagt cttgaagtca tgcgccggtt aaggctaaac tgaaaggaca agttttggtg 9780
   actgcgctcc tccaagccag ttacctcggt tcaaagagtt ggtagctcag agaaccttcg 9840
   aaaaaccgcc ctgcaaggcg gttttttcgt tttcagagca agagattacg cgcagaccaa 9900
   aacgatctca agaagatcat cttattaagg ggtctgacgc tcagtggaac gaaaactcac 9960
   gttaagggat tttggtcatg agattatcaa aaaggatctt cacctagatc cttttaaatt 10020
   aaaaatgaag ttttaaatca atctaaagta tatatgagta aacttggtct gacagttacc 10080
   aatgcttaat cagtgaggca cctatctcag cgatctgtct atttcgttca tccatagttg 10140
   cctgactccc cgtcgtgtag ataactacga tacgggaggg cttaccatct ggccccagtg 10200
   ctgcaatgat accgcgagac ccacgctcac cggctccaga tttatcagca ataaaccagc 10260
   cagccggaag ggccgagcgc agaagtggtc ctgcaacttt atccgcctcc atccagtcta 10320
   ttaattgttg ccgggaagct agagtaagta gttcgccagt taatagtttg cgcaacgttg 10380
   ttgccattgc cgatgataag ctgtcaaaca tggcctgtcg cttgcggtat tcggaatctt 10440
   gcacgccctc gctcaagcct tcgtcactgg tcccgccacc aaacgtttcg gcgagaagca 10500
   ggccattatc gccggcatgg cggccgacgc gcggggagag gcggtttgcg tattgggcgc 10560
   cagggtggtt tttcttttca ccagtgagac gggcaacagc tgattgccct tcaccgcctg 10620
   gccctgagag agttgcagca agcggtccac gctggtttgc cccagcaggc gaaaatcctg 10680
   tttgatggtg gttaacggcg ggatataaca tgagctgtct tcggtatcgt cgtatcccac 10740
   taccgagata tccgcaccaa cgcgcagccc ggactcggta atggcgcgca ttgcgcccag 10800
   cgccatctga tcgttggcaa ccagcatcgc agtgggaacg atgccctcat tcagcatttg 10860
   catggtttgt tgaaaaccgg acatggcact ccagtcgcct tcccgttccg ctatcggctg 10920
   aatttgattg cgagtgagat atttatgcca gccagccaga cgcagacgcg ccgagacaga 10980
   acttaatggg cccgctaaca gcgcgatttg ctggtgaccc aatgcgacca gatgctccac 11040
   gcccagtcgc gtaccgtctt catgggagaa aataatactg ttgatgggtg tctggtcaga 11100
   gacatcaaga aataacgccg gaacattagt gcaggcagct tccacagcaa tggcatcctg 11160
   gtcatccagc ggatagttaa tgatcagccc actgacgcgt tgcgcgagaa gattgtgcac 11220
   cgccgcttta caggcttcga cgccgcttcg ttctaccatc gacaccacca cgctggcacc 11280
   cagttgatcg gcgcgagatt taatcgccgc gacaatttgc gacggcgcgt gcagggccag 11340
   actggaggtg gcaacgccaa tcagcaacga ctgtttgccc gccagttgtt gtgccacgcg 11400
   gttgggaatg taattcagct ccgccatcgc cgcttccact ttttcccgcg ttttcgcaga 11460
   aacgtggctg gcctggttca ccacgcggga aacggtctga taagagacac cggcatactc 11520
   tgcgacatcg tataacgtta ctggtttcac attcaccacc ctgaattgac tctcttccgg 11580
   gcgctatcat gccataccgc gaaaggtttt gcaccattcg atggtgtcaa cgtaaatgca 11640
   tgccgcttcg ccttcgcgcg cgaattgcaa gctgatccgg gcttatcgac tgcacggtgc 11700
   accaatgctt ctggcgtcag gcagccatcg gaagctgtgg tatggctgtg caggtcgtaa 11760
   atcactgcat aattcgtgtc gctcaaggcg cactcccgtt ctggataatg ttttttgcgc 11820
   cgacatcata acggttctgg caaatattct gaaatgagct gttgacaatt aatcatcggc 11880
   tcgtataatg tgtggaattg tgagcggata acaatttcac acaggaaaca gaattaaaag 11940
   atatgaccat gattacgcc 11959
<210> 2
   <211> 31
   <212> DNA
   <213> artificial
<220>
   <223> Primer
<400> 2
   tctagattat tcgtcgtgtt cttcccacgc c 31
<210> 3
   <211> 44
   <212> DNA
   <213> artificial
<220>
   <223> Primer
<400> 3
   gggacgtcga caaggagata tagatgactg aaaaaaaata tatc 44
<210> 4
   <211> 36
   <212> DNA
   <213> artificial
<220>
   <223> Primer
<400> 4
   tctagaaagg agatatagat ggaaaccaaa gatctg 36
<210> 5
   <211> 30
   <212> DNA
   <213> artificial
<220>
   <223> Primer
<400> 5
   gttaattcta gattacgacg ccagcgataa 30
<210> 6
   <211> 40
   <212> DNA
   <213> artificial
<220>
   <223> Primer
<400> 6
   ggacactagt aaggagatat agatgctgcg caccatcctc 40
<210> 7
   <211> 33
   <212> DNA
   <213> artificial
<220>
   <223> Primer
<400> 7
   ctaaaacggg taccctaaat gcttctcgac gtc 33

## Beschreibung

Die vorliegende Erfindung betrifft eine gegenüber ihrem Wildtyp gentechnisch veränderte Zelle, Verfahren zur Herstellung einer gentechnisch veränderten Zelle, die durch diese Verfahren erhältlichen, gentechnisch veränderten Zellen, Verfahren zur Herstellung von 3-Hydroxypropionsäure, ein Verfahren zur Herstellung von Polyacrylaten, ein Verfahren zur Herstellung von Acrylsäureestern sowie die Verwendung von Zellen zur Herstellung von 3-Hydroxypropionsäure.

Acrylsäure ist eine technisch sehr bedeutende Ausgangsverbindung. Sie dient unter anderem zur Herstellung von Polyacrylaten, insbesondere von vernetzten, teilneutralisierten Polyacrylaten, die im trockenen und im wesentlichen wasserfreien Zustand eine große Fähigkeit zur Wasserabsorption aufweisen. Diese als "Superabsorber" bezeichneten, vernetzten Polyacrylate können ein Mehrfaches ihres Eigengewichts an Wasser absorbieren. Aufgrund der hohen Absorptionsfähigkeit eignen sich die absorbierenden Polymere zur Einarbeitung in wasserabsorbierenden Strukturen und Gegenständen, wie z. B. Babywindeln, Inkontinenzprodukten oder Damenbinden. In diesem Zusammenhang wird auf Modern Superabsorbent Polymer Technology; F.L. Buchholz, A.T. Graham, Wiley-VCH, 1998 verwiesen.

Acrylsäureester, wie beispielsweise Methylacrylat und Butylacrylat, sind ebenfalls industriell bedeutende Ausgangsverbindungen, die insbesondere zur Herstellung von Copolymeren eingesetzt werden. Diese Copolymere werden meist in Form von Polymerdispersionen als Klebstoffe, Anstrichmittel oder Textil-, Leder-und Papierhilfsmittel eingesetzt.

Die Herstellung der Acrylsäure erfolgt technisch in erster Linie durch die zweistufige, katalytische

Gasphasenoxidation von Propylen, wobei das Propylen wiederum durch thermische Spaltung von bei der Erdölverarbeitung anfallenden Benzinen erhalten wird. Die auf diese Weise erhaltene Acrylsäure wird anschließend durch den Zusatz von Alkoholen gegebenenfalls verestert.

Nachteilig bei dem zweistufigen Verfahren zur Herstellung von Acrylsäure ist zum einen, dass die in beiden Stufen angewandten Temperaturen zwischen 300 und 450°C zur Bildung von Oligomeren und weiteren unerwünschten Crackprodukten führen. Dieses hat zur Folge, dass eine unerwünscht hohe Menge an höher als die Acrylsäure siedenden Verbindungen oder an Verbindungen, die nur schwer von der Acrylsäure zu trennen sind, wie etwa Essigsäure, anfallen. Diese Verbindungen müssen in der Regel destillativ von der Acrylsäure abgetrennt werden, was wiederum zu einer weiteren thermischen Belastung der Acrylsäure und der damit verbundenen Bildung von Dimeren und Oligomeren führt. Ein hoher Gehalt an Acrylsäuredimeren oder Acrylsäureoligomeren ist jedoch nachteilig, da diese Dimeren oder Oligomeren bei der Herstellung von Superabsorbern durch radikalische Polymerisation von Acrylsäure in Gegenwart von Vernetzern in das Polymer eingebaut werden. Bei der im Anschluss an die Polymerisation erfolgenden Nachbehandlung der Oberfläche der Polymerpartikel, beispielsweise im Rahmen einer Oberflächennachvernetzung, werden die einpolymerisierten Dimere jedoch unter Bildung von β-Hydroxypropionsäure gespalten, die unter den Nachvernetzungsbedingungen unter Bildung von Acrylsäure dehydratisiert wird. Ein hoher Gehalt an dimerer Acrylsäure in der zur Herstellung der Superabsorber eingesetzten Acrylsäure führt daher dazu, dass der Gehalt an Acrylsäuremonomeren bei einer thermischen Behandlung der Polymere, wie sie bei der Nachvernetzung erfolgt, ansteigt.

Auch andere, oftmals toxische Verbindungen sind in der durch die katalytische Gasphasenoxidation erhältlichen Acrylsäure noch nachweisbar. Zu diesen Verunreinigungen gehören insbesondere Aldehyde, die sich störend auf den Polymerisationsverlauf auswirken, mit der Folge, dass die Polymere noch beachtliche Mengen an löslichen Bestandteilen beinhalten.

Zur Lösung dieser Probleme sind im Stand der Technik bereits einige Ansätze beschrieben worden (siehe zum Beispiel EP-A-0 574 260 oder DE-A-101 38 150).

Ein weiterer Nachteil dieses herkömmlichen Verfahrens zur Herstellung von Acrylsäure besteht darin, dass das eingesetzte Edukt (Propylen) aus Erdöl und somit aus nichtnachwachsenden Rohstoffen hergestellt wird, was vor allem im Hinblick auf die zunehmend schwerer und vor allem teurer werdende Erdölgewinnung aus ökonomischen Aspekten vor allem langfristig bedenklich ist.

Auch hier sind im Stand der Technik bereits einige Ansätze beschrieben, um diesem Problem zu begegnen.

So beschreibt WO-A-03/62173 die Herstellung von Acrylsäure, bei dem fermentativ aus Pyruvat zunächst Alpha-Alanin gebildet wird, welches dann mittels des Enzyms 2,3-Aminomutase zu Beta-Alanin umgesetzt wird. Das Beta-Alanin wiederum wird über β-Alanyl-CoA, Acrylyl-CoA, 3-Hydroxypropionyl-CoA oder aber über Malonäuresemialdehyd zu 3-Hydroxypropionsäure umgesetzt, aus der nach einer Dehydratisierung Acrylsäure enthalten wird.

WO-A-02/42418 beschreibt einen weiteren Weg zur Herstellung von beispielsweise 3-Hydroxypropionsäure aus nachwachsenden Rohstoffen. Dabei wird Pyruvat zunächst zu Lactat umgesetzt, aus dem anschließend Lactyl-CoA gebildet wird. Das Lactyl-CoA wird dann über Acrylyl-CoA und 3-Hydroxypropionyl-CoA zu 3-Hydroxypropionsäure umgesetzt. Eine weitere, in der WO-A-02/42418 beschriebene Route zur Herstellung von 3-Hydroxypropionsäure sieht die Umsetzung von Glucose über Propionat, Propionyl-CoA, Acrylyl-CoA und 3-Hydroxypropionyl-CoA vor. Auch beschreibt diese Druckschrift die Umsetzung von Pyruvat zu 3-Hydroxypropionsäure über Acetyl-CoA und Malonyl-CoA. Die durch die jeweiligen Routen erhaltene 3-Hydroxypropionsäure kann durch Dehydratisierung zur Acrylsäure umgesetzt werden.

WO-A-01/16346 beschreibt die fermentative Herstellung von 3-Hydroxypropionsäure aus Glycerol, bei dem Mikroorganismen eingesetzt werden, welche das dhaB-Gen aus *Klebsiella pneumoniae* (ein Gen, welches für Glycerin-Dehydratase codiert) und ein Gen, welches für eine Aldehyd-Dehydrogenase codiert, exprimieren. Auf diese Weise wird aus Glycerin über 3-Hydroxypropionaldehyd 3-Hydroxypropionsäure gebildet, die dann durch Dehydratisierung in Acrylsäure überführt werden kann.

Die EP1083225 offenbart ein Verfahren zur Herstellung von D-Pantothensäure durch Fermentation coryneformer Bakterien, dadurch gekennzeichnet, dass man Bakterien einsetzt, in denen man die für die Pyruvat-Carboxylase (EC-Nummer 6.4.1.1.) codierende Nucleotidsequenz (pyc-Gen) verstärkt, insbesondere überexprimiert.

Straathof et al. in Appl Microbiol Biotechnol. 2005 Jun;67(6):727-34. diskutieren die Möglichkeit der fermentativen Herstellung von Acrylsäure unter Ausnutzung modifizierter Stoffwechselwege rund um beta-Alanin, Methylcitrat und Methylmalonat-CoA.

Der Nachteil der vorstehend beschriebenen fermentativen Verfahren zur Herstellung von 3-Hydroxypropionsäure als Ausgangsverbindungen für die Acrylsäuresynthese besteht unter anderem darin, dass die Menge an gebildeter 3-Hydroxypropionsäure in der Fermentationslösung zu gering ist, um diese Fermentationslösung als Ausgangsmaterial für eine großtechnische Herstellung von Acrylsäure in wirtschaftlich vorteilhafter Weise zu verwenden.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, rekombinante Mikroorganismen bzw. Systeme aus mindestens zwei rekombinanten Mikroorganismen bereitzustellen, die noch besser, insbesondere noch effizienter als die im Stand der Technik beschriebenen Mikroorganismen in der Lage sind, aus nachwachsenden Rohstoffen, insbesondere aus Kohlehydraten und/oder aus Glycerin 3-Hydroxypropionsäure herzustellen, welches anschließend in einer schonenden Dehydratisierungsreaktion in reine Acrylsäure überführt werden kann.

Einen Beitrag zur Lösung der vorstehend genannten Aufgaben leistet ein Verfahren zur Herstellung von 3-Hydroxypropionsäure, umfassend die Verfahrensschritte
i) das in Kontakt bringen einer gegenüber ihrem Wildtyp gentechnisch veränderten Bakterienzelle der Gattungen Corynebacterium oder Escherichia, welche die Eigenschaften a) und b) und c) aufweist:
   a) eine im Vergleich zu ihrem Wildtyp gesteigerte Aktivität eines Enzyms E_{1b}, welches die Umsetzung von Phosphoenolpyruvat zu Oxalacetat katalysiert, wobei das Enzym E_{1b} eine Phosphoenolpyruvat-Carboxylase, welche von dem ppc-Gen kodiert wird, ist,
   b) eine im Vergleich zu ihrem Wildtyp gesteigerte Aktivität eines Enzyms E₂, welches die Umsetzung von Aspartat zu Beta-Alanin katalysiert, und wobei das Enzym E₂ eine Aspartat-Decarboxylase, welche von dem panD-Gen kodiert wird, ist,
   c) die gentechnisch veränderte Zelle ist durch eine Steigerung der Aktivität der
      Transportenzyme ausgewählt aus der Gruppe umfassend
      *multi-drug-resistance-*Proteine wie ebrA und ebrB,
      *multi-drug-efflux* Transporter wie blt- und bmr-Gene,
      vom *E*. *coli* cycA-Gen kodierte Transportsysteme, das hPAT1 Transportsystem,
      das tGAT-B-, rGAT-B-, mGAT3-, mGAT2-,tGAT1 Transportsystem und
      das (B^{0,+}) Transportsystem aus Hase in der Lage, Beta-Alanin aus der Zelle herauszuschleusen,
   mit einem Nährmedium beinhaltend Kohlenhydrate oder Glycerin unter Bedingungen, unter denen aus den Kohlenhydraten oder dem Glycerin Beta-Alanin gebildet wird, welches zumindest teilweise aus der Zelle in das Nährmedium gelangt, so dass ein Beta-Alanin beinhaltendes Nährmedium erhalten wird;
ii) in Kontakt bringen des Beta-Alanin beinhaltenden Nährmediums mit einer weiteren Zelle, welche in der Lage ist, das Beta-Alanin aufzunehmen und zu 3-Hydroxypropionsäure umzusetzen.

In dem erfindungsgemäßen Verfahren eingesetzte Baktereienzelle ist eine gegenüber ihrem Wildtyp gentechnisch veränderte Zelle, welche beide der Eigenschaften a) und b) aufweist:
a) eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % gesteigerte Aktivität eines Enzyms E_{1b}, welches die Umsetzung von Phosphoenolpyruvat zu Oxalacetat katalysiert,
b) eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % gesteigerte Aktivität eines Enzyms E₂, welches die Umsetzung von Aspartat zu Beta-Alanin katalysiert,
   wobei die Zelle neben den Eigenschaften a) und b), durch Eigenschaft c) gekennzeichnet ist:
c) die gentechnisch veränderte Zelle ist in der Lage, Beta-Alanin aus der Zelle herauszuschleusen.

Eine derart gentechnisch veränderte Zelle ist in der Lage, selbst oder in Kombination mit anderen Zellen, welche Beta-Alanin zu 3-Hydroxypropionsäure umsetzen können, aus Kohlehydraten oder aus Glycerin 3-Hydroxypropionsäure zu bilden, da das gebildete Beta-Alanin zu 3-Hydroxypropsionsäure umgesetzt werden kann.

Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Der Begriff "gesteigerte Aktivität eines Enzyms" ist vorzugsweise als gesteigerte intrazelluläre Aktivität zu verstehen. Von der Formulierung "eine gegenüber ihrem Wildtyp gesteigerte Aktivität eines Enzyms" ist insbesondere auch eine Zelle umfasst, deren Wildtyp keine oder zumindest keine nachweisbare Aktivität dieses Enzyms aufweist und die erst nach Erhöhung der Enzymaktivität, beispielsweise durch Überexpression, eine nachweisbare Aktivität dieses Enzyms zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms induziert wird.

Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet oder ein Gen oder Allel nutzt, das für ein entsprechendes Enzym mit einer gesteigerten Aktivität kodiert und gegebenenfalls diese Maßnahmen kombiniert. Erfindungsgemäß eingesetzte gentechnisch veränderte Zellen werden beispielsweise durch Transformation, Transduktion, Konjugation oder einer Kombination dieser Methoden mit einem Vektor erzeugt, der das gewünschte Gen, ein Allel dieses Gens oder Teile davon und einen die Expression des Gens ermöglichenden Vektor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der Enzym-Aktivität in Zellen am Beispiel der Pyruvat-Carboxylase gibt DE-A-100 31 999.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. ((2001) Electrophoresis 22: 1712-1723) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optischer Auswertung mit entsprechender Software zur Konzentrationsbestimmung (Lohaus und Meyer (1989) Biospektrum 5: 32-39; Lottspeich (1999) Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al. (Electophoresis 22: 1712-1723 (2001)), Lohaus et al. (Biospektrum 5: 32-39 (1998)), Lottspeich (Angewandte Chemie 111: 2630-2647 (1999)) und Wilson et al. (Journal of Bacteriology 183: 2151-2155 (2001)) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden gentechnisch veränderte Zellen erhalten. Besonders bevorzugte Mutanten von Enzymen sind insbesondere auch solche Enzyme, die nicht mehr oder zumindest im Vergleich zum Wildtyp-Enzym vermindert *feedback-*inhibierbar sind.

Wird die Erhöhung der Enzymaktivität durch Erhöhung der Expression eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Desweiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138: 35-41 (1994)), Tsuchiya und Morinaga (Bio/Technology 6: 428-430 (1988)), bei Eikmanns et al. (Gene 102: 93-98 (1991)), in EP-A-0 472 869, im US 4,601,893, bei Schwarzer und Pühler (Bio/Technology 9: 84-87 (1991)), bei Reinscheid et al. (Applied and Environmental Microbiology 60: 126-132 (1994)), bei LaBarre et al. (Journal of Bacteriology 175: 1001-1007 (1993)), in WO-A-96/15246, bei Malumbres et al. (Gene 134: 15-24 (1993)), in JP-A-10-229891, bei Jensen und Hammer (Biotechnology and Bioengineering 58: 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Zur Erhöhung der Expression der jeweiligen Gene werden zum Beispiel episomale Plasmide eingesetzt. Als Plasmide eignen sich insbesondere solche, die in coryneformen Bakterien repliziert werden. Zahlreiche bekannte Plasmidvektoren, wie zum Beispiel pZ1 (Menkel et al., Applied and Environmental Microbiology 64: 549-554 (1989)), pEKEx1 (Eikmanns et al., Gene 107: 69-74 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie zum Beispiel solche, die auf pCG4 (US 4,489,160) oder pNG2 (Serwold-Davis et al., FEMS Microbiology Letters 66: 119-124 (1990)) oder pAG1 (US 5,158,891) beruhen, können in gleicher Weise eingesetzt werden.

Weiterhin eignen sich auch solche Plasmidevektoren, mit Hilfe derer man das Verfahren der Genamplifikation durch Integration in das Chromosom anwenden kann, so wie es beispielsweise von Reinscheid et al. (Applied and Environmental Microbiology 60: 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise *Escherichia coli*), nicht aber in *Corynebacterium glutamicum* repliziert werden kann. Als Vektoren kommen beispielsweise pSUP301 (Simon et al., Bio/Technology 1: 784-791 (1983)), pK18mob oder pK19mob (Schäfer et al., Gene 145: 69-73 (1994)), pGEM-T (Promega Corporation, Madison, Wisconsin, USA), pCR2.1-TOPO (Shuman, Journal of Biological Chemistry 269: 32678-84 (1994)), pCR®Blunt (Invitrogen, Groningen, Niederlande), pEM1 (Schrumpf et al., Journal of Bacteriology 173: 4510-4516)) oder pBGS8 (Spratt et al., Gene 41: 337-342 (1986)) in Frage. Der Plasmidvektor, der das zu amplifizierende Gen enthält, wird anschließend durch Konjugation oder Transformation in den gewünschten Stamm von *Corynebacterium glutamicum* überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al. (Applied Microbiology and Biotechnology 29: 356-362 (1988)), Dunican und Shivnan (Bio/Technology 7: 1067-1070 (1989)) und Tauch et al. (FEMS Microbiology Letters 123: 343-347 (1994)) beschrieben. Nach homologer Rekombination mittels eines "cross-over"-Ereignisses enthält der resultierende Stamm mindestens zwei Kopien des betreffenden Gens.

Bei den erfindungsgemäß eingesetzten Zellen handelt es sich um gentechnisch veränderte Zellen.

Erfindungsgemäß eingesetzte Zellen sind diejenigen der Gattungen Corynebacterium und Escherichia, wobei Escherichia coli, Corynebacterium glutamicum und Corynebacterium efficiens besonders bevorzugt und Corynebacterium glutamicum am meisten bevorzugt ist bzw. sind.

Bei dem Enzym E1b handelt es sich um eine Phosphoenolpyruvat-Carboxylase (EC 4.1.1.31), welche die Umsetzung von Phosphoenolypyruvat zu Oxalacetat katalysiert. Erfindungsgemäß eingesetzte Phosphoenolpyruvat-Carboxylase ist diejenige Phosphoenolpyruvat-Carboxylase, die von dem Gen ppc kodiert wird. ppc-Gene für Wildtyp-Phosphoenolpyruvat-Caboxylasen oder Mutanten dieser Enzyme sind beispielsweise aus US 6,599,732, US 5,573,945, US 4,757,009 sowie aus US 4,980,285 bekannt. Die Herstellung von Zellen mit erhöhter Phosphoenolpyruvat-Carboxylase-Aktivität ist unter anderem in US 4,757,009 beschrieben. Neben der Überexpression des Enzyms können auch die Expression von Enzymmutanten angewendet werden. In diesem Zusammenhang besonders bevorzugte Mutanten sind diejenigen, die keiner Aktivierung durch Acetyl-CoA bedürfen und/oder die feedback-inhibiert sind im Hinblick auf Asparaginsäure (siehe hierzu insbesondere US 6,919,190). Grundsätzlich können auch im Zusammenhang mit der Phosphoenolpyruvat-Carboxylase die entsprechenden Gene jeder nur denkbaren Herkunft, gleichgültig ob aus Bakterien, Hefen, Pflanzen, Tieren oder Pilzen, verwendet werden. Weiterhin können auch hier alle Allele des ppc-Gens verwendet werden, insbesondere auch diejenigen, die sich aus der Degeneriertheit des genetischen Codes oder durch funktionsneutrale Sinnmutationen (sense mutations) ergeben

Die Bestimmung der Aktivität der Phosphoenolpyruvat-Carboxylase erfolgt vorzugsweise gemäß dem in der Dissertation von Petra Peters-Wendisch am Forschungszentrum Jülich GmbH "Anaplerotische Reaktionen in Corynebacterium glutamicum: Untersuchung zur Bedeutung der PEP-Carboxylase und der Pyruvat-Carboxylase im Zentralstoffwechsel und bei der Aminosäureproduktion" (1996) beschriebenen Verfahren.

Einen Überblick über Phosphoenolpyruvat-Carboxylasen und Pyruvat-Carboxylasen geben insbesondere auch Sauer und Eikmanns (FEMS Microbiology Reviews 29: 765-794 (2005)).

Bei dem Enzym E₂ handelt es sich Aspartat-Decarboxylase, welche von dem panD-Gen kodiert wird. Die Aspartat-Decarboxylase katalysiert die Umsetzung von Aspartat zu Beta-Alanin. Gene für die Aspartat-Decarboxylase (panD-Gene) unter anderem aus Escherichia coli (FEMS Microbiology Letters 143: 247-252 (1996)), Photorhabdus luminescens subsp. laumondii, Mycobacterium bovis subsp. bovis sowie aus zahlreichen anderen Mikroorganismen wurden bereits kloniert und sequenziert. Insbesondere die Nukleotidsequenz des panD-Gens aus Corynebacterium glutamicum ist in der DE-A-198 55 313 beschrieben. Grundsätzlich können panD-Gene jeder nur denkbaren Herkunft, gleichgültig ob aus Bakterien, Hefen, Pflanzen, Tieren oder Pilzen, verwendet werden. Weiterhin können alle Allele des panD-Gens verwendet werden, insbesondere auch diejenigen, die sich aus der Degeneriertheit des genetischen Codes oder durch funktionsneutrale Sinnmutationen (*sense mutations*) ergeben.

Eine neben der Aspartat-Decarboxylase aus Corynebacterium glutamicum erfindungsgemäß besonders bevorzugte Aspartat-Decarboxylase ist die Escherichia coli-Mutante DV9 (Vallari und Rock, Journal of Bacteriology 164: 136-142 (1985)).

Die Herstellung von Zellen mit erhöhter Aspartat-Decarboxylase-Aktivität ist unter anderem in DE-A-198 55 314 eingehend beschrieben. Erfindungsgemäß besonders bevorzugt eingesetzte Zellen sind solche, die vorstehend beschriebene Enzymmutante DV9 aus Escherichia coli oder aber panD aus Corynebacterium glutamicum aufweisen bzw. solche, welche DNA-Sequenzen aufweisen, die für eines dieser Enzym kodieren und die dieses Enzym in ausreichender Menge exprimieren.

Die Bestimmung der Aspartat-Decarboxylase-Aktivität erfolgt gemäß dem von Dusch et al. (Applied and Environmental Microbiology 65(4): 1530-1539 (1999)) in dem Kapitel mit der Überschrift "Aspartate decarboxylase activity assay" beschriebenen Testverfahren.

Die erfindungsgemäß eingsetzten Zellen sind des weiteren dadurch gekennzeichnet, dass sie neben den Eigenschaften a) und b), durch Eigenschaft c) gekennzeichnet ist:
c) die gentechnisch veränderte Zelle ist in der Lage, Beta-Alanin aus der Zelle herauszuschleusen.

Der Begriff "3-Hydroxypropionsäure", wie er hierin verwendet wird, umfasst die protonierte Form der 3-Hydroxypropionsäure ebenso wie die deprotonierte Form der 3-Hydroxypropionsäure (= 3-Hydroxypropionat) und Mischungen aus protonierter und deprotonierter Form. Der Begriff "herausschleusen" umfasst sowohl den aktiven als auch den passiven Transport von Beta-Alanin aus der Zelle hinaus in das die Zelle umgebende Medium.

Erfindungsgemäß eingesetzte Zellen, welche die Bedingung c) erfüllen, sind dadurch gekennzeichnet, dass sie endogene und/oder exogene, vorzugsweise exogene Transportenzyme in der Zellmembran aufweisen, welche selektiv oder nicht selektiv, vorzugsweise selektiv, aktiv oder passiv, gegebenenfalls im Austausch gegen oder zusammen mit Ionen wie Natrium, Kalium oder Chlor Beta-Alanin durch die Zellmembran hindurch nach außen, also in den Bereich außerhalb der Zelle, schleusen können (=Efflux des Beta-Alanins aus der Zelle). Dabei ist es erfindungsgemäß besonders bevorzugt, dass die gentechnisch veränderte Zelle einen im Vergleich zu ihrem Wildtyp erhöhten, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % erhöhten Efflux von Beta-Alanin aus der Zelle heraus aufweist. Ein um 10 % erhöhter Efflux bedeutet dabei, dass die gentechnisch veränderte Zelle im Vergleich zu ihrem Wildtyp unter gleichen Bedingungen, insbesondere bei gleicher intrazellulärer und extrazellulärer Beta-Alanin-Konzentration in einem definierten Zeitintervall um 10 % mehr Beta-Alanin aus der Zelle herauszuschleusen vermag. Vorzugsweise wird der erhöhte Efflux durch eine Steigerung der Aktivität der vorstehend genannten Transportenzyme realisiert, wobei die Erhöhung wiederum durch die bereits im Zusammenhang mit den Enzymen E_{1b} und E₂ genannten Techniken (Mutation des Transportenzyms oder Steigerung der Genexpression des Transportenzyms) erfolgen kann.

In diesem Zusammenhang eingesetzte Transportenzyme sind die sogenannten "*multi-drug-resistance*"-Proteine (MDR-Proteine), beispielsweise mit den Genen ebrA und ebrB sowie die sogenannten "*multi-drug-efflux transporter*", wobei die *"multi-drug-efflux transporter*", beispielsweise mit den blt- und bmr-Genen besonders bevorzugt sind. Geeignete Transportsysteme für Beta-Alanin sind auch in "Handbook of Corynebacterium glutamicum", Herausgeber L. Eggeling und M. Bott, CRC Press, Boca Raton, USA, 2005, Kapitel IV, "Genomic Analyses of Transporter Proteins in Corynebacterium glutamicum and Corynebacterium efficiens", B. Winnen, J. Felce, und M. H. Saier, Jr., Seiten 149-186 beschrieben. Weitere geeignete Transportsysteme für Beta-Alanin, insbesondere solche, die vom cycA-Gen kodiert werden, sind in Schneider et al (Appl. Microbiol. Biotechnol. 65(5): 576-582 (2004)) beschrieben. Geeignete Transportsysteme für Beta-Alanin sind weiterhin in Anderson und Thwaites (J. Cell. Physiol. 204(2): 604-613 (2005)), Brechtel und King (Biochem. J. 333: 565-571 (1998)), Guimbal et al. (Eur. J. Biochem. 234(3): 794-800 (1995)), Munck und Munck (Biochim. Biophys. Acta 1235(1): 93-99 (1995)) und Shuttleworth und Goldstein (J. Exp. Zool. 231(1): 39-44 (1984)) beschrieben.

Erfindungsgemäß eingesetzte Zellen, die in der Lage sind, Beta-Alanin aufzunehmen und zur 3-Hydroxypropionsäure umzusetzen" sind in der Lage, das gebildete Beta-Alanin in 3-Hydroxypropionsäure umzusetzen. Dabei sind grundsätzlich mindestens zwei Varianten denkbar.

Gemäß der Variante A können die Zellen das Beta-Alanin über Beta-Alanyl-CoA, Acrylyl-CoA und Hydroxypropionyl-CoA zu 3-Hydroxypropionsäure umsetzen. In diesem Zusammenhang ist es besonders bevorzugt, dass die Zelle eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % gesteigerte Aktivität mindestens eines, vorzugsweise aller folgenden Enzyme E₃ bis E₆ aufweist:
- eines Enzyms E₃, welches die Umsetzung von Beta-Alanin zu Beta-Alanyl-Coenzym A katalysiert,
- eines Enzyms E₄, welches die Umsetzung von Beta-Alanyl-Coenzym A zu Acrylyl-Coenzym A katalysiert,
- eines Enzyms E₅, welches die Umsetzung von Acrylyl-Coenzym A zu 3-Hydroxypropionyl-Coenzym A katalysiert,
- eines Enzyms E₆, welches die Umsetzung von 3-Hydroxypropionyl-Coenzym A zu 3-Hydroxypropionsäure katalysiert.

Erfindungsgemäß besonders bevorzugt eingesetzte gentechnisch veränderte Zellen sind dabei diejenigen, bei denen, gegebenenfalls zusätzlich zur Steigerung der Enzymaktivitäten E_{1b} sowie E₂ die Aktivität folgender Enzyme oder Enzymkombinationen gesteigert ist: E₃, E₄, E₅, E₆, E₃E₄, E₃E₅, E₃E₆, E₄E₅, E₄E₆, E₅E₆, E₃E₄E₅, E₃E₄E₆, E₃E₅E₅, E₄E₅E₆ oder E₃E₄E₅E₆.

Die Steigerung der enzymatischen Aktivität der Enzyme E₃ bis E₆ kann auch hier durch die im Zusammenhang mit den Enzymen E_{1b} und E₂ genannten Techniken, wie Mutation oder Erhöhung der Enzymexpression, erfolgen.

Es ist in diesem Zusammenhang weiterhin bevorzugt, dass das Enzym
E₃ eine Coenzym A-Transferase (EC 2.8.3.1) oder Coenzym A-Synthetase, vorzugsweise eine Coenzym A-Transferase,
E₄ eine Beta-Alanyl-Coenzym A-Ammonium-Lysase (EC 4.3.1.6),
E₅ eine 3-Hydroxypropionyl-Coenzym A-Dehydratase (EC 4.2.1.-, insbeson-dere EC 4.2.1.17) und
E₆ eine Coenzym A-Transferase (EC 2.8.3.1), 3-Hydroxypropionyl-Coen-zym A-Hydrolase (EC 3.1. 2. -) oder 3-Hydroxybutyryl-Coenzym A-Hydro-lase (EC 3.1.2.4), vorzugsweise eine Coenzym A-Transferase ist.

Bevorzugte Enzyme mit einer CoA-Transferaseaktivität sind diejenigen aus Megasphaera elsdenii, Clostridium propionicum, Clostridium kluyveri und auch aus Escherichia coli. Als Beispiele für eine CoA-Transferase codierende DNA-Sequenz seien an dieser Stelle die in der WO-A-03/062173 mit der SEQ ID NO: 24 bezeichnete Sequenz aus Megasphaera elsdenii genannt. Des weiteren bevorzugte Enzyme sind diejenigen Varianten der CoA-Transferase, die in der WO-A-03/062173 beschrieben werden.

Geeignete Enzyme mit einer Beta-Alanyl-Coenzym A-Ammonium-Lyase-Aktivität sind beispielsweise diejenigen aus Clostridium propionicum. DNA-Sequenzen, welche für ein solches Enzym kodieren, können beispielsweise aus Clostridium propionicum, wie im Beispiel 10 der WO-A-03/062173 beschrieben, erhalten werden. Die DNA-Sequenz, welche für die Beta-Alanyl-Coenzym A-Ammonium-Lyase aus Clostridium propionicum kodiert, ist in der WO-A-03/062173 als SEQ ID NO: 22 angegeben.

Geeignete Enzyme mit einer 3-Hydroxypropionyl-Coenzym A-Dehydratase-Aktivität sind insbesondere diejenigen Enzyme, die von Genen ausgewählt aus der Gruppe umfassend ECHS1, EHHADH, HADHA, CG4389, CG6543, CG6984, CG8778, ech-1, ech-2, ech-3, ech-5, ech-6, ech-7, FCAALL.314, FCAALL.21, FOX2, ECI12, ECI1, paaF, paaG, yfcX, fadB, faoA, fadB1x, phaB, echA9, echA17, fad-1, fad-2, fad-3, paaB, echA7, dcaE, hcaA, RSp0671, RSp0035, RSp0648, RSp0647, RS03234, RS03271, RS04421, RS04419, RS02820, RS02946, paaG2, paaG1, ech, badK, crt, ydbS, eccH2, pimF, paaG3, fabJ-1, caiD-2, fabJ-2, ysiB, yngF, yusL, phaA, phaB, fucA, caiD, ysiB, echA3, echA5, echA6, echA7, echA8, echA14, echA15, echA16, echA17, echA18.1, echA19, echA20, echA21, echA2, echA4, echA9, echA11, echA10, echA12, echA13, echA18, echA1, fadB-1, echA8-1, echA12-2, fadB-2, echA16-2, CgI0919, fadB1, SCF41.23, SCD10.16, SCK13.22, SCP8.07c, StBAC16H6.14, SC5F2A.15, SC6A5.38, faoA, hbd1, crt, hbd-1, hbd-2, hbd-5, fad-4, hbd-10, fad-5, hbd1, paaF-1, paaF-2, paaF-3, paaF-4, paaF-5, paaF-6 und paaF-7 kodiert werden. Als Beispiele für erfindungsgemäß geeignete 3-Hydroxypropionyl-Coenzym A-Dehydratasen seien insbesondere diejenigen aus Chloroflexus aurantiacus, Candida rugosa, Rhodosprillium rubrum und Rhodobacter capsulates genannt. Ein besonderes Beispiel einer für eine 3-Hydroxypropionyl-Coenzym A-Dehydratase kodierenden DNA-Sequenz ist beispielsweise in der WO-A-02/42418 als SEQ ID NO: 40 angegeben.

Die Herstellung gentechnisch veränderter Zellen, in denen mindestens eine, vorzugsweise alle der vorstehend genannten enzymatischen Aktivitäten E₃ bis E₆ erhöht worden ist bzw. sind, ist beispielsweise in den Beispielen der WO-A-02/42418 und der WO-A-03/062173 beschrieben.

Gemäß der Variante B können die Zellen das Beta-Alanin über Malonsäuresemialdehyd zu 3-Hydroxypropionsäure umsetzen. In diesem Zusammenhang ist es besonders bevorzugt, dass die Zelle eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % gesteigerte Aktivität mindestens eines der, vorzugsweise beider Enzyme E7 und E8 aufweist:
- eines Enzyms E₇, welches die Umsetzung von Beta-Alanin zu Malon-säuresemialdehyd katalysiert,
- eines Enzyms E₈, welches die Umsetzung von Malonsäuresemialdehyd zu 3-Hydroxypropionsäure katalysiert.

Erfindungsgemäß besonders bevorzugt eingesetzte gentechnisch veränderte Zellen sind dabei diejenigen, bei denen, gegebenenfalls zusätzlich zur Steigerung der Enzymaktivitäten E_{1b} sowie E₂ die Aktivität folgender Enzyme oder Enzymkombinationen gesteigert ist: E₇, E₈ und E₇E₈.

Die Steigerung der enzymatischen Aktivität der Enzyme E₇ und E₈ kann auch hier durch die im Zusammenhang mit dem Enzym E_{1b} und E₂ genannten Techniken wie Mutation oder Erhöhung der Enzymexpression erfolgen.

Es ist in diesem Zusammenhang weiterhin bevorzugt, dass das Enzym
- E₇: eine Beta-Alanin-2-Oxoglutarat-Aminotransferase (EC 2.6.1.19) oder eine Taurin-2-Oxoglutarat-Transaminase (2.6.1.55), vorzugsweise eine Beta-Alanin-2-Oxoglutarat-Aminotransferase, und
- E₈: eine 3-Hydroxypropionat-Dehydrogenase (EC 1.1.1.59) oder 3-Hydroxy-butyrat-Dehydrogenase (EC 1.1.1.30), vorzugsweise jedoch eine 3-Hydroxypropionat-Dehydrogenase (EC 1.1.1.59)
ist.

Die Gene der Beta-Alanin-2-Oxoglutarat-Aminotransferase sind beispielsweise aus Neurospora crassa bekannt ("Über die beta-Alanin-alpha-Ketoglutarat-Transaminase", Aurich und Hoppe-Seyler's, Z. Physiol. Chem. 326: 25-33 (1961)). Weitere Informationen zu Genen dieses Enzyms aus anderen Mikroorganismen können insbesondere der KEGG GENE Database (KEGG = Kyoto Encyclopedia of Genes and Genomes) entnommen werden. Auch die Gene der 3-Hydroxypropionat-Dehydrogenase oder der 3-Hydroxybutyrat-Dehydrogenase aus unterschiedlichsten Mikroorganismen können der KEGG GENE Database entnommen werden.

Die erfindungsgemäß eingesetzten Zellen sind in der Lage, in Kombination mit anderen Mikroorganismen, welche 3-Hydroxypropionsäure aus Beta-Alanin herstellen können aus Pyruvat 3-Hydroxypropionsäure zu bilden.

Ausgehend vom Pyruvat als Startpunkt für die Herstellung des Beta-Alanins sind nun wiederum zwei unterschiedliche Ausführungsformen der erfindungsgemäß eingesetzten Zellen denkbar.

Gemäß der ersten Ausführungsform der erfindungsgemäß eingestzten Zellen sind diese in der Lage, das für die Herstellung des Beta-Alanins benötigte Pyruvat insbesondere auch aus Glycerin als Kohlenstoffquelle herzustellen.

In diesem Zusammenhang ist es besonders bevorzugt, dass die erfindungsgemäß eingesetzte Zelle eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 % gesteigerte Aktivität mindestens eines, vorzugsweise aller folgenden Enzyme E₉ bis E₂₂ aufweist:
- eines Enzyms E₉, welches die Diffusion von Glycerin in die Zelle hinein erleichtert,
- eines Enzyms E₁₀, welches die Umsetzung von Glycerin zu Glycerin-3-Phosphat katalysiert,
- eines Enzyms E₁₁, welches die Umsetzung von Glycerin-3-Phosphat zu Dihydroxyacetonphosphat katalysiert,
- eines Enzyms E₁₂, welches die Übertragung von Schwefel auf den Schwefelsakzeptor Thioredoxin 1 katalysiert,
- eines Enzyms E₁₃, welches die Hydrolyse von Phospholipiden unter Bildung von Alkoholen und Glycerin katalysiert,
- eines Enzyms E₁₄, welches den Transport von Glycerin-3-Phosphat in die Zelle hinein im Austausch gegen Phosphat katalysiert;
- eines Enzyms E₁₅, welches die Umsetzung von Dihydroxyacetonphosphat zu Glycerinaldehyd-3-Phosphat katalysiert,
- eines Enzyms E₁₆, welches die Umsetzung von Glycerinaldehyd-3-Phosphat zu 1,3-Biphosphoglycerat katalysiert,
- eines Enzyms E₁₇, welches die Umsetzung von 1,3-Biphosphoglycerat zu 3-Phosphoglycerat katalysiert,
- eines Enzyms E₁₈, welches die Umsetzung von 3-Phosphoglycerat zu 2-Phosphoglycerat katalysiert,
- eines Enzyms E₁₉, welches die Umsetzung von 2-Phosphoglycerat zu Phosphoenolpyruvat katalysiert,
- eines Enzyms E₂₀, welches die Umsetzung von Phosphoenolpyruvat zu Pyruvat katalysiert,
- eines Enzyms E₂₁, welches die Umsetzung von Glycerin zu Dihydroxyaceton katalysiert,
- eines Enzyms E₂₂, welches die Umsetzung von Dihydroxyaceton zu Dihydroxyacetonphosphat katalysiert.

Erfindungsgemäß besonders bevorzugt eingesetzte gentechnisch veränderte Zellen sind dabei diejenigen, bei denen, gegebenenfalls zusätzlich zur Steigerung der Enzymaktivitäten E_{1b} sowie E₂ und gegebenenfalls einer oder mehrere der Enzymaktivitäten E₃ bis E₆ oder E₇ und E₈ die Aktivität folgender Enzyme oder Enzymkombinationen gesteigert ist: E₉, E₁₀, E₁₁, E₁₂, E₁₃, E₁₄, E₁₅, E₁₆, E_{17,} E₁₈, E₁₉, E₂₀, E₂₁, E₂₂, E₁₀E₁₁, wobei eine Erhöhung einer oder mehrere der Enzymaktivitäten ausgewählt aus der Gruppe bestehend aus E₉, E₁₀, E₁₁, E₁₃, E₁₄, E₂₁ und E₂₂ besonders bevorzugt ist und eine Erhöhung der Enzymaktivitäten E₁₀ und E₁₁ am meisten bevorzugt ist.

In diesem Zusammenhang ist es insbesondere bevorzugt, dass das Enzym
- E₉: ein Aquaglyceroporin (Glycerol-Facilitator), der vorzugsweise vom glpF-Gen kodiert wird,
- E₁₀: eine Glycerin-Kinase (EC 2.7.1.30), die vorzugsweise vom glpK-Gen kodiert wird,
- E₁₁: eine Glycerin-3-Phosphat-Dehydrogenase (EC 1.1.99.5), vorzugsweise eine FAD-abhängige Glycerin-3-Phosphat-Dehydrogenase, wobei die Glycerin-3-Phosphat-Dehydrogenase vorzugsweise von dem glpA-Gen, dem glpB-Gen, dem glpC-Gen oder dem glpD-Gen, besonders bevorzugt dem glpD-Gen kodiert wird,
- E₁₂: eine Schwefeltransferase, welche von dem glpE-Gen kodiert wird,
- E₁₃: eine Glycerinphosphodiesterase (EC 3.1.4.46), welche vorzugsweise vom glpQ-Gen kodiert wird,
- E₁₄: eine Glycerin-3-Phosphat-Permease, welche vorzugsweise vom glpT-Gen kodiert wird
- E₁₅: eine Triosephosphat-Isomerase (EC 5.3.1.1),
- E₁₆: eine Glyceraldehyd-3-Phosphat-Deydrogenase (EC 1.2.1.12),
- E₁₇: eine Phosphoglycerat-Kinase (EC 2.7.2.3),
- E18: eine Phosphoglycerat-Mutase (EC 5.4.2.1),
- E19: eine Enolase (EC 4.2.1.11),
- E₂₀: eine Pyruvat-Kinase (EC 2.7.1.40)
- E₂₁: eine Glycerin-Dehydrogenase (EC 1.1.1.6), die vorzugsweise vom gldA-Gen kodiert wird, und
- E₂₂: eine Dihydroxyaceton-Kinase (EC 2.7.1.29), die vorzugsweise vom dhaK-Gen kodiert wird,
ist.

Die Gensequenzen der vorstehend genannten Enzyme sind in der Literatur bekannt und können zum Beispiel der KEGG GENE Database, den Datenbanken des National Center for Biotechnology Information (NCBI) der National Library of Medicine (Bethesda, MD, USA) oder der Nukleotidsequenz-Datenbank der European Molecular Biologies Laboratories (EMBL, Heidelberg, Deutschland bzw. Cambridge, UK) entnommen werden. Des Weiteren sind das Glyceraldehyd-3-Phosphat-Dehydrogenase codierende gap-Gen (Eikmanns (1992), Journal of Bacteriology 174: 6076-6086), das für die Triosephosphat-Isomerase codierende tpi-Gen (Eikmanns (1992), Journal of Bacteriology 174: 6076-6086) und das für die 3-Phosphoglycerat-Kinase codierende pgk-Gen (Eikmanns (1992), Journal of Bacteriology 174: 6076-6086) aus anderen Quellen bekannt.

Mit den bekannten Genen der Enzyme E₉ bis E₂₂ lassen sich gentechnisch veränderte Zellen, in denen mindestens eine, besonders bevorzugt mindestens zwei, darüber hinaus bevorzugt mindestens drei und am meisten bevorzugt alle Aktivitäten der Enzyme E₉ bis E₂₂ durch die vorstehend im Zusammenhang mit dem Enzym E_{1b} und E₂ beschriebenen Techniken (Mutation des Enzyms oder Steigerung der Enzymexpression) erhöht worden ist, herstellen. Diese Zellen sind in der Lage, in Gegenwart von Glycerin als einziger Kohlenstoffquelle (oder aber zusammen mit Kohlehydraten als weitere Kohlenstoffquelle) kultiviert werden zu können.

Neben der Erhöhung einer oder mehrerer der Enzymaktivitäten E₉ bis E₂₂ kann es in diesem Zusammenhang auch vorteilhaft sein, wenn in den erfindungsgemäß eingesetzten Zellen folgende Gene vorzugsweise heterolog exprimieren:
- das glpG-Gen oder das b3424-Gen,
- das gIpX-Gen oder das 3925-Gen"
- das dhaR-Gen, das ycgU-Gen oder das b1201-Gen
- das fsa-Gen, das mipB-Gen, das ybiZ-Gen oder das B0825-Gen
- das talC-Gen, das fsaB-Gen, das yijG-Gen oder das b3946-Gen.

Die Nukleotidsequenzen dieser Gene können wiederum der KEGG GENE Database, den Datenbanken des National Center for Biotechnology Information (NCBI) der National Library of Medicine (Bethesda, MD, USA) oder der Nukleotidsequenz-Datenbank der European Molecular Biologies Laboratories (EMBL, Heidelberg, Deutschland bzw. Cambridge, UK) entnommen werden.

Gemäß der zweiten Ausführungsform der erfindungsgemäß eingesetzten Zellen sind diese in der Lage, das für die Herstellung des Beta-Alanins benötigte Pyruvat nicht oder zumindest nur in geringem Umfang aus Glycerin zu erhalten. In diesem Falle erfolgt die Bereitstellung des Pyruvates in den Zellen vornehmlich durch die Glykolyse. Derartige Zellen sind in einem Nährmedium kultivierbar, welches Kohlenhydrate wie beispielsweise Glukose als Kohlenstoffquelle beinhaltet.

In diesem Falle ist es bevorzugt, dass die erfindungsgemäß eingesetzten Zellen, gegebenenfalls neben einer erhöhten Aktivität mindestens eines, vorzugsweise aller vorstehend genannten Enzyme E₉ bis E₂₂, eine im Vergleich zu ihrem Wildtyp gesteigerte, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis 5.000 %, besonders bevorzugt bis 2.500 %gesteigerte Aktivität mindestens eines, vorzugsweise aller folgenden Enzyme E₁₆ bis E₂₀ und E₂₃ bis E₂₇ aufweisen:
- eines Enzyms E₂₃, welches die Diffusion von Glycerin in die Zelle hinein erleichtert,
- eines Enzyms E₂₄, welches die Umsetzung von Glukose zu α-D-Glukose-6-Phosphat katalysiert,
- eines Enzyms E₂₅, welches die Umsetzung von α-D-Glukose-6-Phosphat zu β-D-Fruktose-6-Phosphat katalysiert,
- eines Enzyms E₂₆, welches die Umsetzung von β-D-Fruktose-6-Phosphat zu β-D-Fruktose-1,6-Biphosphat katalysiert,
- eines Enzyms E₂₇, welches die Umsetzung von β-D-Fruktose-1,6-Biphosphat zu Glycerinaldehyd-3-Phosphat und Dihydroxyacetonphosphat katalysiert,
- eines Enzyms E₁₆, welches die Umsetzung von Glycerinaldehyd-3-Phosphat zu 1,3-Biphosphoglycerat katalysiert,
- eines Enzyms E₁₇, welches die Umsetzung von 1,3-Biphosphoglycerat zu 3-Phosphoglycerat katalysiert,
- eines Enzyms E₁₈, welches die Umsetzung von 3-Phosphoglycerat zu 2-Phosphoglycerat katalysiert,
- eines Enzyms E₁₉, welches die Umsetzung von 2-Phosphoglycerat zu Phosphoenolpyruvat katalysiert, und
- eines Enzyms E₂₀, welches die Umsetzung von Phosphoenolpyruvat zu Pyruvat katalysier.

Erfindungsgemäß besonders bevorzugt eingestzte gentechnisch veränderte Zellen sind dabei diejenigen, bei denen, gegebenenfalls zusätzlich zur Steigerung der Enzymaktivitäten E_{1b} sowie E₂ und gegebenenfalls einer oder mehrere der Enzymaktivitäten E₃ bis E₆ oder E₇ und E₈ die Aktivität folgender Enzyme oder Enzymkombinationen gesteigert ist: E₁₆, E₁₇, E₁₈, E₁₉, E₂₀, E₂₃, E₂₄, E₂₅, D₂₆, E₂₇ und E₁₆E₁₇E₁₈E₁₉E₂₀E₂₃E₂₄E₂₅E₂₆E₂₇.

In diesem Zusammenhang ist es insbesondere bevorzugt, dass das Enzym
- E₂₃: ein Glukosetransporter, vorzugsweise ein durch ein Gen ausgewählt aus der Gruppe umfassend glutl, gluP oder fucP kodierter Glukose-Transporter, oder eine Glukose-Permease (2.7.1.69),
- E₂₄: eine Glukokinase (2.7.1.2),
- E₂₅: eine Glukose-6-Phosphat-Isomerase (EC 5.3.1.9),
- E₂₆: eine 6-Phosphofrukto-Kinase (EC 2.7.1.11),
- E₂₇: eine Fruktose-Bisphosphat-Aldolase (EC 4.1.2.13),
- E₁₆: eine Glyceraldehyd-3-Phosphat-Deydrogenase (EC 1.2.1.12),
- E₁₇: eine Phosphoglycerat-Kinase (EC 2.7.2.3),
- E₁₈: eine Phosphoglycerat-Mutase (EC 5.4.2.1),
- E₁₉: eine Enolase (EC 4.2.1.11), und
- E₂₀: eine Pyruvat-Kinase (EC 2.7.1.40)
ist.

Die Nukleotidsequenzen dieser Gene können wiederum der KEGG GENE Database, den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) entnommen werden.

Weiterhin ist es in Zusammenhang mit dieser zweiten Ausführungsform der erfindungsgemäß eingesetzten Zelle bevorzugt, dass neben den vorstehend genannten Enzymen E_{1b} und/oder E₂ sowie gegebenenfalls mindestens eines der Enzyme E₃ bis E₆, E₇ oder E₈ sowie E₁₂ bis E₂₇ auch die Aktivität von Enzymen des Phosphotransferase-Systems erhöht worden ist. In diesem Zusammenhang ist es insbesondere bevorzugt, dass in den erfindungsgemäß eingesetzten Zellen die ptsI- und ptsM-Gene verstärkt, vorzugsweise überexprimiert sind. In diesem Zusammenhang wird insbesondere auf US 6,680,187 und 6,818,432 verwiesen.

In den erfindungsgemäß eingesetzten Zellen kann weiterhin, unabhängig davon, ob sie Glycerin oder Glukose als primäre Nährstoffquelle verwenden und auch unabhängig davon, ob sie alleine oder in Kombination mit anderen Zellen über Malonsäuresemialdehyd oder über Beta-Alanyl-Coenzym A, Acrylyl-Coenzym A und 3-Hydroxypropionyl-Coenzym A 3-Hydroxypropionsäure bilden, die Aktivität der Aspartat-Aminotransferase (EC 2.6.1.1.A) erhöht sein. Die Sequenz des entsprechenden Gens (aspB) kann unter anderem der KEGG GENE Database entnommen werden.

Weiterhin ist es in den erfindungsgemäß eingesetzten Zellen bevorzugt, die Aktivität von Enzymen,
- E₂₈: welche die Umsetzung von Oxaloacetat zu Phosphoenolpyruvat katalysieren, wie etwa die Phosphoenylpyruvat-Carboxy-Kinase (EC 4.1.1.49) (siehe auch DE-A-199 50 409),
- E₂₉: welche die Umsetzung von Pyruvat zu Acetat katalysieren, wie etwa die Pyruvat-Oxidase (EC 1.2.2.2) (siehe auch DE-A-199 51 975),
- E₃₀: welche die Umsetzung von α-D-Glukose-6-Phosphat zu β-D-Fruktose-6-Phosphat katalysieren (siehe auch US 09/396,478),
- E₃₁: welche die Umsetzung von Beta-Alanin zu Carnosin katalysieren, wie etwa die Carnosin-Synthase (EC 6.3.2.11),
- E₃₂: welche die Umsetzung von Beta-Alanin zu Alpha-Alanin katalysieren, wie etwa die Alanin-Aminomutase,
- E₃₃: welche die Umsetzung von Beta-Alanin zu (R)-Pantothenat katalysieren, wie etwa die Pantothenat-Beta-Alanin-Ligase (EC 6.3.2.1),
- E₃₄: welche die Umsetzung von Beta-Alanin zu N-Carbomyl-Beta-Alanin katalysieren, wie etwa die Beta-Ureidopropionase (EC 3.5.1.6),
- E₃₅: welche die Umsetzung von Pyruvat zu Lactat katalysieren, wie etwa die l-Lactat-Dehydrogenase (EC 1.1.1.27) oder die Lactat-Malat-Transhydro-genase (EC 1.1.99.7),
- E₃₆: welche die Umsetzung von Pyruvat zu Acetyl-Coenzym A katalysieren, wie etwa die Pyruvat-Dehydrogenase (EC 1.2.1.51),
- E₃₇: welche die Umsetzung von Pyruvat zu Acetylphosphat katalysiert, wie etwa die Pyruvat-Oxidase (EC 1.2.3.3),
- E₃₈: welche die Umsetzung von Pyruvat zu Acetat katalysiert, wie etwa die Pyruvat-Dehydrogenase (EC 1.2.2.2),
- E₃₉: welche die Umsetzung von Pyruvat zu Phosphoenolpyruvat katalysieren, wie etwa die Phosphoenolpyruvat-Synthase (EC 2.7.9.2) oder die Pyruvat-Phosphat-Dikinase (EC 2.7.9.1), und/oder
- E₄₀: welche die Umsetzung von Pyruvat zu Alanin katalysieren, wie etwa die Alanin-Transaminase (2.6.1.2) oder die Alanin-Oxosäure-Transaminase (EC 2.6.1.12)
abzuschwächen, vorzugsweise um mindestens 10 %, besonders bevorzugt um mindestens 25 %, darüber hinaus bevorzugt um mindestens 50 %, darüber hinaus noch mehr bevorzugt um mindestens 75 %, des weiteren bevorzugt um mindesten 100 % und am meisten bevorzugt um mindestens 500 %, maximal vorzugsweise bis maximal 5.000 %, besonders bis maximal 2.500 % abzuschwächen, wobei die Abschwächung der Enzyme E₂₈, E₃₀, E₃₃, E₃₅, E₃₆ und E₄₀ besonders bevorzugt ist.

Der Begriff "abschwächen" beschreibt in diesem Zusammenhang die Verringerung oder Ausschaltung der intrazellulären Aktivität eines oder mehrerer Enzyme in einer Zelle, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym inaktiviert und gegebenenfalls diese Maßnahmen kombiniert.

Insbesondere kann es gemäß einer besonderen Ausführungsform der erfindungsgemäß eingesetzten Zellen sinnvoll sein, gezielt den Pentosephosphat-Weg zu fördern, beispielsweise durch Erhöhen der Aktivität der Glucose-6-Phosphat-Dehydrogenase (EC 1.1.1.49) und der 6-Phosphogluconat-Dehydrogenase (EC 1.1.1.44), und gleichzeitig die Glykolyse zu hemmen, beispielsweise durch Abschwächung der Aktivität der Glucose-6-Phosphat-Isomerase, wie dieses in WO-A-01/07626 beschrieben ist.

Gemäß einer besonderen Ausführungsform der erfindungsgemäß eingesetzten Zelle ist bei dieser die Aktivität der Glutamat-Dehydrogenase (EC 1.4.1.4) durch eine der im Zusammenhang mit dem Enzym E_{1b} und E₂ genannten Techniken erhöht. Die Gene dieses Enzyms aus zahlreichen Mikroorganismen können ebenfalls der KEGG GENE Database entnommen werden. Weiterhin werden in US 6,355,454 und in WO-A-00/53726 Gene der Glutamat-Dehydrogenase und Möglichkeiten zur Überexpression dieses Enzyms beschrieben.

Weiterhin ist es bei den erfindungsgemäß eingesetzten, gentechnisch veränderten Zellen bevorzugt, dass diese Beta-Alanin und Alpha-Alanin im Gewichtsverhältnis von mindestens 2 _{:} 1, besonders bevorzugt mindestens 3 _{:} 1 und darüber hinaus bevorzugt mindestens 4 _{:} 1 bilden. Eine Bildung von Beta-Alanin und Alpha-Alanin im Gewichtsverhältnis von mindestens 2 _{:} 1 bedeutet dabei, dass die Zellen innerhalb eines Zeitintervalls von 29 Stunden bei 37°C mindestens doppelt soviel Beta-Alanin wie Alpha-Alanin bilden, vorzugsweise bilden und in das die Zellen umgebende Nährmedium freisetzen.

In diesem Zusammenhang ist es besonders bevorzug, dass bei diesen erfindungsgemäß eingesetzten Zellen
- die Aktivität der Glutamat-Dehydrogenase (EC 1.4.1.4) erhöht ist, und
- die Aktivität der Pyruvat-Carboxylase (EC 6.4.1.1) erhöht ist, und
- die Aktivität der Aspartat-Decarboxylase (EC 4.1.1.11) erhöht ist, und
- die Aktivität der Glucose-6-Phosphat-Isomerase
   (EC 5.3.1.9) abgeschwächt ist.

Weiterhin betrifft die vorliegende Erfindung insbesondere den Einsatz einer gentechnisch veränderten Zelle, welche
- erhöhte Phosphoenolpyruvat-Carboxylase-Aktivität (EC 4.1.1.31) und
- erhöhte Aspartat-Decarboxylase-Aktivität (EC 4.1.1.11), vorzugsweise durch eine Asparat-Decarboxylase aus *Corynebacterium glutamicum*
und mindestens eines der, vorzugsweise alle nachfolgenden Eigenschaften aufweist:
- erhöhte Beta-Alanin-2-Oxoglutarat-Aminotransferase-Aktivität (EC 2.6.1.19), und
- erhöhte 3-Hydroxypropionat-Dehydrogenase-Aktivität (EC 1.1.1.59).

Als Zellen, die in der Lage sind, Beta-Alanin aufzunehmen und zur 3-Hydroxypropionsäure umzusetzen, sind insbesondere diejenigen Zellen bevorzugt, in denen die Aktivität mindestens eines, vorzugsweise aller Enzyme E₃ bis E₆ oder aber Zellen, in denen die Aktivität mindestens eines, vorzugsweise beider Enzyme E₇ und E₈ gegenüber dem Wildtyp erhöht worden ist bevorzugt. Solche Zellen sind beispielsweise in der WO-A-02/42418 und der WO-A-03/62173 beschrieben. Besonders bevorzugt sind darüber hinaus Zellen, bei denen zusätzlich zu diesen enzymatischen Aktivitäten auch die Aktivität von Enzymen erhöht worden ist, welche den Transport bzw. Efflux von Beta-Alanin in die Zellen hinein erhöhen. In diesem Zusammenhang ist insbesondere der GABA-Transporter GAT-2 sowie das durch das cycA-Gen kodierte Transportsystem, das in Schneider et al., (Appl. Microbiol. Biotechnol. 65: 576-582 (2004)) beschrieben wird, bevorzugt.

Die erfindungsgemäß eingesetzten, gentechnisch veränderten Zellen können im Verfahrensschritt i) kontinuierlich oder diskontinuierlich im batch-Verfahren (Satzkultivierung) oder im fed-batch-Verfahren (Zulaufverfahren) oder repeated-fed-batch-Verfahren (repetitives Zulaufverfahren) zum Zwecke der Produktion von Beta-Alanin mit dem Nährmedium in Kontakt und somit kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Besonders bevorzugt ist der Einsatz von Kohlehydraten, insbesondere Monosaccharide, Oligosaccharide oder Polysaccharide, wie dies in US 6,01,494 und US 6,136,576 beschrieben ist, von C₅-Zuckern oder von Glycerin.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natrium(hydrogen)carbonat, Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Insbesondere bei der Verwendung von Zellen, welche Glycerin als Substrat umwandeln können, kann es bevorzugt sein, als Zellen solche Zellen einzusetzen, die in der US 6,803,218 beschrieben werden und in diesen Zellen die Aktivität der Enzyme E_{1b} und E₂ zu erhöhen (sowie gegebenenfalls der weiteren Enzyme E₃ bis E₂₀) . In diesem Fall können die Zellen bei Temperaturen im Bereich von 40 bis 100°C kultiviert werden.

Zeitgleich mit der Bildung von Beta-Alanin oder von diesem Verfahrenschritt getrennt wird das Beta-Alanin beinhaltenden Nährmedium mit den weiteren Zellen, welche in der Lage sind, das Beta-Alanin aufzunehmen und zu 3-Hydroxypropionsäure umzusetzen, in Kontakt gebracht. Dabei können die erfindungsgemäß eingesetzten Zellen und die weiteren Zellen gemeinsam in einem Nährmedium kultiviert werden, so dass das von den erfindungsgemäß eingesetzten Zellen abgegebene Beta-Alanin quasi *in statu nascendii* von den weiteren Zellen aufgenommen und zu 3-Hydroxypropionsäure umgesetzt wird. Denkbar ist aber auch, zunächst ein Beta-Alanin-haltiges Nährmedium zu bilden, aus diesem die erfindungsgemäß eingesetzten Zellen abzutrennen und erst dann dieses Beta-Alanin enthaltende Nährmedium mit den weiteren Zellen in Kontakt zu bringen. Besonders bevorzugt ist jedoch die gleichzeitige Kultivierung der erfindungsgemäß eingesetzten und der weiteren Zellen.

Das erfindungsgemäße Verfahren zur Herstellung von 3-Hydroxypropionsäure kann als weiteren Verfahrensschritt iii) auch die Aufreinigung der letztendlich erhaltenen 3-Hydroxypropionsäure aus dem Nährmedium umfassen. Diese Aufreinigung kann durch jedes dem Fachmann bekannte Reinigungsverfahren erfolgen. So können beispielsweise Sedimentations-, Filtrations- oder Zentrifugationsverfahren eingesetzt werden, um die Zellen abzutrennen. Aus dem von Zellen befreiten, 3-Hydroxypropionsäure-haltigen Nährmedium kann die 3-Hydroxypropionsäure durch Extraktion, Destillation, Ionen-Austausch, Elektrodialyse oder Kristalisation isoliert werden.

Die Aufreinigung der 3-Hydroxypropionsäure aus der Nährlösung erfolgt gemäß einer besonderen Ausgestaltung des erfindungsgemäßen Verfahrens kontinuierlich, wobei es in diesem Zusammenhang weiterhin bevorzugt ist, auch die Fermentation kontinuierlich durchzuführen, so dass der gesamte Prozess von der enzymatischen Umsetzung der Edukte unter Bildung von 3-Hydroxypropionsäure bis zur Aufreinigung der 3-Hydroxypropionsäure aus dem Nährmedium kontinuierlich durchgeführt werden kann. Zur kontinuierlichen Aufreinigung der 3-Hydroxypropionsäure aus dem Nährmedium wird diese kontinuierlich über eine Vorrichtung zur Abtrennung der bei der Fermentation eingesetzten Zellen, vorzugsweise über einen Filter mit einer Ausschlussgröße in einem Bereich von 20 bis 200 kDa geführt, in dem eine Fest-/Flüssig-Trennung stattfindet. Denkbar ist auch der Einsatz einer Zentrifuge, einer geeigneten Sedimentationsvorrichtung oder eine Kombination dieser Vorrichtungen, wobei es besonders bevorzugt ist, zumindest einen Teil der Zellen zunächst durch Sedimentation abzutrennen und anschließend das von den Zellen teilweise befreite Nährmedium einer Ultrafiltrations- oder Zentrifugationsvorrichtung zuzuführen.

Das hinsichtlich seines 3-Hydroxypropionsäure-Anteils angereicherte Fermentationserzeugnis wird nach der Abtrennung der Zellen einer vorzugsweise mehrstufigen Trennanlage zugeführt. In dieser Trennanlage sind mehrere hintereinander geschaltete Trennstufen vorgesehen, aus denen jeweils Rückführ-Leitungen ausmünden, die zum zweiten Fermentationstank zurückgeführt sind. Weiterhin führen aus den jeweiligen Trennstufen Ableitungen heraus. Die einzelnen Trennstufen können nach dem Prinzip der Elektrodialyse, der Umkehrosmose, der Ultrafiltration oder der Nanofiltration arbeiten. In der Regel handelt es sich um Membran-Trenneinrichtungen in den einzelnen Trennstufen. Die Auswahl der einzelnen Trennstufen ergibt sich aus Art und Umfang der Fermentations-Nebenprodukte und Substratreste.

Neben der Abtrennung der 3-Hydroxypropionsäure mittels Elektrodialyse, Umkehrosmose, Ultrafiltration oder Nanofiltration, in deren Verlauf als Endprodukt eine wässrige 3-Hydroxypropionsäure-Lösung erhalten wird, kann die 3-Hydroxypropionsäure auch durch Extraktionsverfahren aus dem von Zellen befreiten Nährmedium abgetrennt werden, wobei in diesem Fall letztendlich die reine 3-Hydroxypropionsäure erhalten werden kann. Zur Abtrennung der 3-Hydroxypropionsäure durch Extraktion können dem Nährmedium, in dem die 3-Hydroxypropionsäure als Ammoniumsalz vorliegt, beispielsweise hochsiedende organische Amine zugesetzt werden. Anschließend wird die so erhaltene Mischung erhitzt, wobei Ammoniak und Wasser entweichen und die 3-Hydroxypropionsäure in die organische Phase extrahiert wird. Dieses als "Salt-Splitting" bezeichnete Verfahren ist der WO-A-02/090312 zu entnehmen.

Weiterhin leistet ein Verfahren zur Herstellung von Acrylsäure einen Beitrag zur Lösung der eingangs genannten Aufgaben, umfassend die Verfahrensschritte
I) Herstellung von 3-Hydroxypropionsäure durch die vorstehend beschriebenen Verfahren, gegebenenfalls gefolgt von einem oder mehreren Aufreinigungsschritten,
II) Dehydratisierung der 3-Hydroxypropionsäure unter Bildung von Acrylsäure.

Die Dehydratisierung der 3-Hydroxypropionsäure kann grundsätzlich in flüssiger Phase oder in der Gasphase durchgeführt werden, wobei eine Flüssigphasen-Dehydratisierung bevorzugt ist. Weiterhin ist es erfindungsgemäß bevorzugt, dass die Dehydratisierung in Gegenwart eines Katalysators erfolgt, wobei die Art des eingesetzten Katalysators davon abhängig ist, ob eine Gasphasen- oder eine Flüssigphasenreaktion durchgeführt wird. Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Im Anschluss an die Dehydratisierung wird eine Acrylsäure beinhaltende Phase erhalten, die gegebenenfalls durch weitere Aufreinigungsschritte, insbesondere durch Destillationsverfahren, Extraktionsverfahren oder Kristallisationsverfahren oder aber durch eine Kombination dieser Verfahren aufgereinigt werden kann.

Einen weiteren Beitrag zur Lösung der eingangs genannten Aufgaben leistet auch ein Verfahren zur Herstellung von Polyacrylaten, umfassend die Verfahrensschritte
I) Herstellung von 3-Hydroxypropionsäure durch eines der vorstehend beschriebenen Verfahren, gegebenenfalls gefolgt von einem oder mehreren Aufreinigungsschritten,
II) Dehydratisierung der 3-Hydroxypropionsäure unter Bildung von Acrylsäure gemäß dem vorstehend beschrieben Verfahren, gegebenenfalls gefolgt von einem oder mehreren Aufreinigungsschritten,
III) Radikalische Polymerisation der Acrylsäure.

Die radikalische Polymerisation der Acrylsäure erfolgt durch dem Fachmann bekannte Polymerisationsverfahren und kann sowohl in einer Emulsion oder Suspension als auch in wässriger Lösung durchgeführt werden. Weiterhin können bei der Polymerisation weitere Co-Monomere, insbesondere Vernetzer zugegen sein. Besonders bevorzugt ist die radikalische Polymerisation der im Verfahrensschritt II) erhaltenen Acrylsäure in zumindest teilneutralisierter Form in Gegenwart von Vernetzern. Bei dieser Polymerisation werden Hydrogele erhalten, die dann zerkleinert, gemahlen und gegebenenfalls Oberflächenmodifiziert, insbesondere Oberflächennachvernetzt werden können. Die so erhaltenen Polymere eignen sich insbesondere zum Einsatz als Superabsorber in Hygieneartikeln wie etwa Windeln oder Damenbinden.

Einen Beitrag zur Lösung der eingangs genannten Aufgaben leistet auch ein Verfahren zur Herstellung von Acrylsäureestern, umfassend die Verfahrensschritte
I) Herstellung von 3-Hydroxypropionsäure durch eines der vorstehend beschriebenen Verfahren, gegebenenfalls gefolgt von einem oder mehreren Aufreinigungsschritten,
II) Dehydratisierung der 3-Hydroxypropionsäure unter Bildung von Acrylsäure gemäß dem vorstehend beschrieben Verfahren, gegebenenfalls gefolgt von einem oder mehreren Aufreinigungsschritten,
III) Veresterung der Acrylsäure.

Die Veresterung der Acrylsäure erfolgt durch dem Fachmann bekannte Veresterungsverfahren, besonders bevorzugt dadurch, dass die im Verfahrensschritt II) erhaltene Acrylsäure mit Alkoholen, vorzugsweise mit Methanol, Ethanol, 1-Propanol, 2-Propanol, n-Butanol, tert.-Butanol oder iso-Butanol in Kontakt gebracht und auf eine Temperatur von mindestens 50°C, besonders bevorzugt mindestens 100°C erhitzt wird. Gegebenenfalls kann das bei der Veresterung gebildete Wasser durch den Zusatz geeigneter Trennhilfen mittels azeotroper Destillation aus dem Reaktionsgemisch entfernt werden.

Die vorliegende Erfindung wird nun anhand nichtlimitierender Figuren und Beispiele näher erläutert.

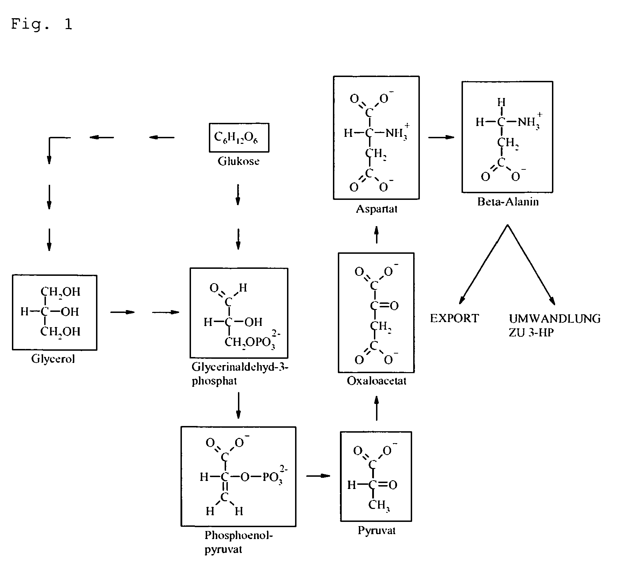

Es zeigt die Figur 1 das Reaktionsschema zur Bildung von Beta-Alanin aus Glukose oder Glycerin über Pyruvat, Oxaloacetat und Aspartat.

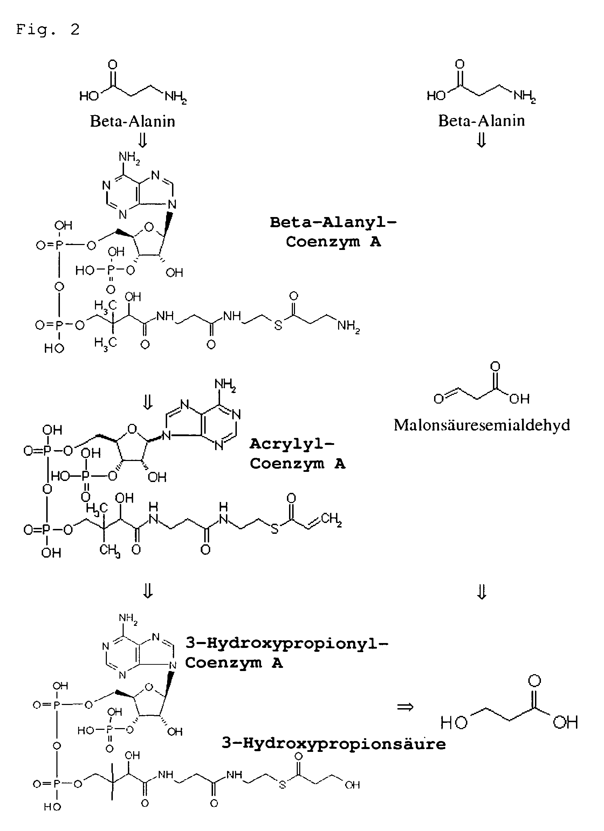

Es zeigt die Figur 2 das Reaktionsschema zur Bildung von 3-Hydroxypropionsäure aus Beta-Alanin über Beta-Alanyl-Coenzym A, Acrylyl-Coenzym A und 3-Hydroxypropionyl-Coenzym A oder über Malonsäuresemialdehyd.

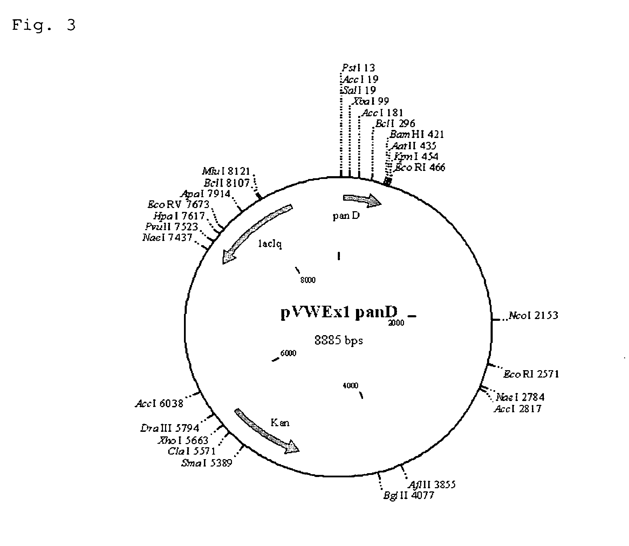

Es zeigt die Figur 3 den Plasmidvektoren pVWex1-panD.

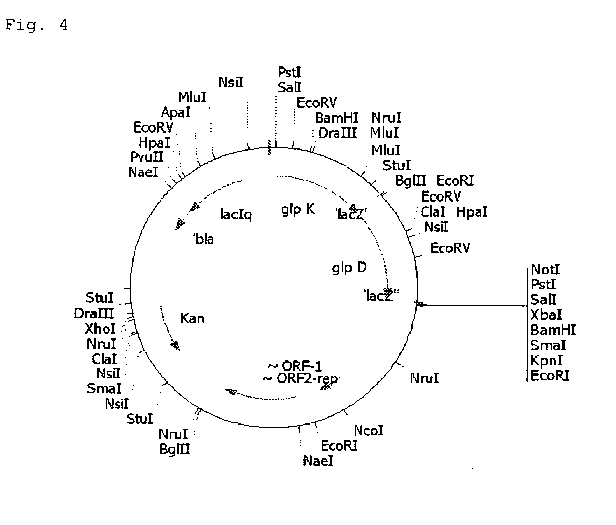

Es zeigt die Figur 4 den Plasmidvektoren pVWEx1-glpKD_{E.c.}.

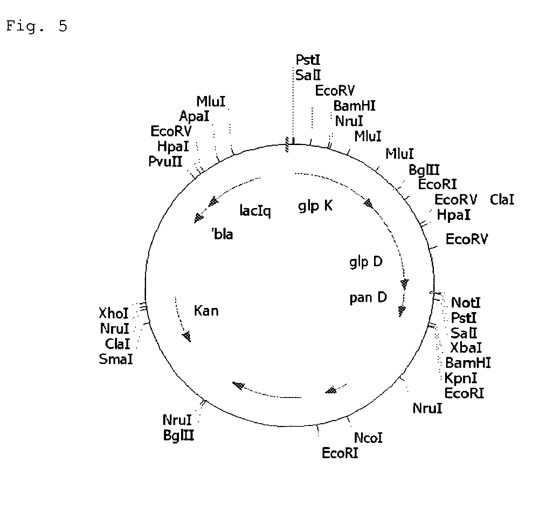

Es zeigt die Figur 5 den Plasmidvektoren pVWEx1-panD-glpKD_{E.c.}.

### Beispiel

Es wurde eine gentechnisch veränderte Zelle des Stammes Corynebacterium glutamicum hergestellt, in der die heterologen Gene glpK, glpD, und die homologen Gene pyc und panD exprimiert wurden. Dabei wurde wie folgt vorgegangen:

Als Ausgangsstämme wurden der Wildtypstamm ATCC13032 (hinterlegt bei der Deutsche Sammlung von Mikroorganismen und Zellkulturen GmbH, Braunschweig mit der DSM-Nummer 20300) und der Stamm DM1727 verwendet. Der Stamm DM1727 wurde von Georgi et al. (Metabolic Engineering 7: 291-301 (2005)) beschrieben und stellt einen gentechnisch veränderten Corynebacterium glutamicum-Stamm dar, der eine gegenüber dem Wildtyp-Stamm erhöhte Aktivität der Pyruvat-Carboxylase aufweist. Diese erhöhte Aktivität des Enzyms ist auf eine Mutation der Aminosäure an Position 458 (Austausch von Prolin durch Serin) zurückzuführen. Siehe hierzu auch "A novel methodology employing Corynebacterium glutamicum genome information to generate a new L-lysineproducing mutant" (Ohnishi et al., Applied Microbiology and Biotechnology 58: 217-223 (2002)).

### 1. Herstellen der Plasmidvektoren

Zunächst wurden die nachfolgenden zwei PCR-Primer synthetisiert (Unterstrichenes Nukleotid entspricht dem Basenpaar im *E. coli*-Genom MG1655 (GenBank Reference Sequence NC 000913 (U00096), Schnittstellen sind fett gedruckt):
- glpKᵣₑᵥ:: 5'**TCTAGA**TTATTCGTCGTGTTCTTCCCACGCC (SEQ. ID NO. 2) (das unterstrichene Nukleotid entspricht dem Basenpaar 4113737 im MG1655-Genom, die Schnittstelle entspricht einer XbaI-Schnittstelle)
- glpK_{for}:: 5'GGGAC**GTCGAC***AAGGAGATATAG*ATGACTGAAAAAAAATATATC (SEQ. ID NO. 3) (das unterstrichene Nukleotid entspricht dem Basenpaar 4115245 im MG1655-Genom, die Schnittstelle entspricht einer SalI-Schnittstelle)

Die Primer entsprachen den Basen 4113737 bis 4113762 und 4115225 bis 4115245 des glpK- Gens von *E*. *coli.* Mit diesen Primern konnte mittels PCR nach der Standardmethode von Innis et al. (PCR protocols. A guide to methods and applications, 1990, Academic Press) für nicht-degenerierte, homologe Primer ein Fragment von 1533 Basenpaaren chromosomaler DNA von E. coli, die, wie bei Eikmanns et al. (Microbiology, 140: 1817-1828 (1994)) beschrieben, isoliert wurde, amplifiziert werden.

Dieses PCR-Fragment wurde in den Plasmidvektor pGEM-T (Promega Corporation, Madison, Wisconsin, USA) unter Erhalt des Plasmidvektors pGEM-T-glpK_{E.c.} kloniert.

Im Anschluss daran wurden die nachfolgenden zwei PCR-Primer synthetisiert (unterstrichenes Nukleotid entspricht dem Basenpaar im E. coli-Genom MG1655, Schnittstellen sind fett gedruckt und kursive Bereich markierten eine Ribosomenbindungstelle):
- glpD_{for}:: 5'**TCTAGA***AAGGAGATATAG*ATGGAAACCAAAGATCTG (SEQ. ID NO. 4) (das unterstrichene Nukleotid entspricht dem Basenpaar 3560036 im MG1655-Genom, die Schnittstelle entspricht einer XbaI-Schnittstelle)
- glpDᵣₑᵥ:: 5'GTTAAT**TCTAGA**TTACGACGCCAGCGATAA (SEQ. ID NO. 5) (das unterstrichene Nukleotid entspricht dem Basenpaar 3561541 im MG1655-Genom, die Schnittstelle entspricht einer XbaI-Schnittstelle)

Die Primer entsprachen den Basen 3560036 bis 3560053 und 3561524 bis 3561541 des plpD- Gens von *E*. *coli.* Mit diesen Primern konnte mittels PCR nach der Standardmethode von Innis et al. (PCR protocols. A guide to methods and applications, 1990, Academic Press) für nicht-degenerierte, homologe Primer ein Fragment von 1524 Basenpaaren chromosomaler DNA von E. coli, die, wie bei Eikmanns et al. (Microbiology 140: 1817-1828 (1994)) beschrieben, isoliert wurde, amplifiziert werden.

Dieses PCR-Fragment wurde in den Plasmidvektor pGEM-T (Promega Corporation, Madison, Wisconsin, USA) unter Erhalt des Plasmidvektors pGEM-T-glpD_{E.c.} kloniert.

Anschließend wurde das 1524-Basenpaar-Fragment mit XbalI aus dem Plasmidvektor pGEM-T glpD_{E.c.} herausgeschnitten und in den mit SpeI-SAP (SAP = Shrimp Alkaline Phosphatase) geschnittenen Plasmidvektor pGEM-T-glpK_{E.c.} unter Erhalt des Plasmidvektors pGEM-T-glpKD_{E.c} kloniert. Anschließend wurde das glpKD_{E.c} -Fragment mittels SalI herausgeschnitten und in mit SalI geschnittenem Plasmidvektor pVWEx1 (dieses Expressionsplasmid wurde durch Peters-Wendisch et al., Journal of Molecular Microbiology and Biotechnology 3: 295-300 (2001) beschrieben) unter Erhalt des Plasmidvektors pVWEx1-glpKD_{E.c.} kloniert.

Im Anschluss daran wurden die nachfolgenden zwei PCR-Primer synthetisiert (unterstrichenes Nukleotid entspricht dem Basenpaar im *Corynebacterium glutamicum* -Genom (NC003450), Schnittstellen sind fett gedruckt und kursive Bereiche markieren eine Ribosomenbindungstelle):
- NCg10133_{for}:: 5'GGAC**ACTAGT**AAGGAGATATAGATGCTGCGCACCATCCTC (SEQ. ID NO. 6) (das unterstrichene Nukleotid entspricht dem Basenpaar 145570 im Genom von *Corynebacterium glutamicum,* die Schnittstelle entspricht einer SpeI-Schnittstelle)
- NCgl0133ᵣₑᵥ:: 5'CTAAAACG**GGTACC**CTAAATGCTTCTCGACGTC (SEQ. ID NO. 7) (das unterstrichene Nukleotid entspricht dem Basenpaar 147980 im Genom von Corynebacterium glutamicum, die Schnittstelle entspricht einer KpnI-Schnittstelle)

Die Primer entsprachen den Basen 147570 bis 147588 und 147964 bis 147980 des panD-Gens von *C*. *glutamicum.* Mit diesen Primern konnte mittels PCR nach der Standardmethode von Innis et al. (PCR protocols. A guide to methods and applications, 1990, Academic Press) für nicht-degenerierte, homologe Primer ein Fragment von etwa 430 Basenpaaren chromosomaler DNA von *C*. *glutamicum,* die, wie bei Eikmanns et al. (Microbiology 140: 1817-1828 (1994)) beschrieben, isoliert wurde, amplifiziert werden.

Dieses PCR-Fragment wurde in den Plasmidvektor pGEM-T (Promega Corporation, Madison, Wisconsin, USA) unter Erhalt des Plasmidvektors pGEM-T-panD kloniert.

Anschließend wurde das panD-Fragment mittels SpeI und KpnI aus pGEM-T-panD herausgeschnitten und in den mit XbaI und KpnI geschnittenen Plasmidvektor pVWEx1 unter Erhalt des Plasmidvektors pVWEx1-panD kloniert. Des Weiteren wurde das mittels SpeI und KpnI aus pGEM-T-pand geschnittene panD-Fragment in mit XbaI und KpnI geschnittenen Plasmidvektor pVWEx1-glpKD_{E.c.} unter Erhalt des Plasmidvektors pVWEx1-panD-glpKD_{E.c.} (SEQ. ID NO. 1) kloniert.

Die Plasmidvektoren pVWEx1-panD, pVWEx1-glpKD_{E.c.} und pVWEx1-panD-glpKD_{E.c.} (SEQ. ID NO. 1) sind in den Figuren 3 bis 5 gezeigt.

### 2. Tranformation der Zellen

In die eingangs genannten Ausgangsstämme wurden die Expressionsplasmide pVWEx1, pVWEx1-panD_{c.g.}, **pVWEx1-**glpKD_{E.c.} und pVWEx1-glpKD_{E.c.}panD_{C.g.} mittels Elektroporation eingebracht (nach van der Rest et al., Appl. Microbiol. Biotechnol. 52: 541-545 (1999)).

### 3. Kultivierung der Zellen

Mit Glukose als Kohlenstoffquelle

Die mit diesen Plasmiden transformierten Stämme wurden in CGXII-Medium kultiviert, welches von Georgi et al. (Metabolic Engineering 7: 291-301 (2005)) und von Marx et al. (US 6,355,454) beschrieben wurde. Das Medium enthielt 40 g/kg Glukose.

Die Alpha- bzw. Beta-Alanin-Konzentration wurde mittels HPLC nachgewiesen. Die Methode wurde von Georgi et al. (Metabolic Engineering 7: 291-301 (2005)) und von Marx et al. (US 6,355,454) beschrieben. Zur Identifizierung des Alpha-Alanin bzw. Beta-Alanin-Signals wurde ein entsprechender Standart eingesetzt.

Nach 29-stündiger Kultivierung der Zellen wurden folgenden Konzentrationen an Alpha-Alanin und Beta-Alanin im Nährmedium gemessen:

| C. glutamicum-Stamm | Alpha-Alanin | Beta-Alanin |
|---|---|---|
| ATCC13032 (pVWEx1) | 20,4 mM | < 1 mM |
| ATCC13032 (pVWEx1-panD_{C.g.} ) | 15,5 mM | 23,9 mM |
| DM1727 (pVWEx1-panD_{C.g.} ) | 5,0 mM | 18,6 mM |

### 3.2. Mit Glycerin als Kohlenstoffquelle

Die mit diesen Plasmiden transformierten Stämme wurden in CGXII-Medium kultiviert, welches von Georgi et al. (Metabolic Engineering 7: 291-301 (2005)) und von Marx et al. (US 6,355,454) beschrieben wurde. Das Medium enthielt 9 g/kg Glycerin.

Die Alpha- bzw. Beta-Alanin-Konzentration wurde mittels HPLC nachgewiesen. Die Methode wurde von Georgi et al. (Metabolic Engineering 7: 291-301 (2005)) und von Marx et al. (US 6,355,454) beschrieben. Zur Identifizierung des Alpha-Alanin- bzw. Beta-Alanin-Signals wurde ein entsprechender Standard eingesetzt.

Nach 24-stündiger Kultivierung der Zellen wurden folgende Konzentrationen an Alpha-Alanin und Beta-Alanin im Nährmedium gemessen:

| C. glutamicum-Stamm | Alpha-Alanin | Beta-Alanin |
|---|---|---|
| ATCC13032 (pVWEx1-glpKD_{E.c.}) | 2,8 mM | < 1 mM |
| ATCC13032 (pVWEx1-glpKD_{E.c.}panD_{C.g.}) | 3 mM | 0,5 mM |
| DM1727 (pVWEx1-glpKD_{E.c.}panD_{C.g.}) | 0,4 mM | 0,5 mM |

## Patentansprüche

1. Ein Verfahren zur Herstellung von 3-Hydroxypropionsäure, umfassend die Verfahrensschritte
i) das in Kontakt bringen einer gegenüber ihrem Wildtyp gentechnisch veränderten Bakterienzelle der Gattungen Corynebacterium oder Escherichia, welche die Eigenschaften a) und b) und c) aufweist:
a) eine im Vergleich zu ihrem Wildtyp gesteigerte Aktivität eines Enzyms E_{1b}, welches die Umsetzung von Phosphoenolpyruvat zu Oxalacetat katalysiert, wobei das Enzym E_{1b} eine Phosphoenolpyruvat-Carboxylase, welche von dem ppc-Gen kodiert wird, ist,
b) eine im Vergleich zu ihrem Wildtyp gesteigerte Aktivität eines Enzyms E₂, welches die Umsetzung von Aspartat zu Beta-Alanin katalysiert, und wobei das Enzym E₂ eine Aspartat-Decarboxylase, welche von dem panD-Gen kodiert wird, ist,
c) die gentechnisch veränderte Zelle ist durch eine Steigerung der Aktivität der Transportenzyme ausgewählt aus der Gruppe umfassend
*multi-drug-resistance-*Proteine wie ebrA und ebrB,
*multi-drug-efflux* Transporter wie blt- und bmr-Gene,
vom *E*. *coli* cycA-Gen kodierte Transportsysteme, das hPAT1 Transportsystem,
das tGAT-B-, rGAT-B-, mGAT3-, mGAT2-,tGAT1 Transportsystem und
das (B^{0,+}) Transportsystem aus Hase in der Lage, Beta-Alanin aus der Zelle herauszuschleusen,
mit einem Nährmedium beinhaltend Kohlenhydrate oder Glycerin unter Bedingungen, unter denen aus den Kohlenhydraten oder dem Glycerin Beta-Alanin gebildet wird, welches zumindest teilweise aus der Zelle in das Nährmedium gelangt, so dass ein Beta-Alanin beinhaltendes Nährmedium erhalten wird;
ii) in Kontakt bringen des Beta-Alanin beinhaltenden Nährmediums mit einer weiteren Zelle, welche in der Lage ist, das Beta-Alanin aufzunehmen und zu 3-Hydroxypropionsäure umzusetzen.

2. Ein Verfahren zur Herstellung von Acrylsäure, umfassend die Verfahrensschritte
I) Herstellung von 3-Hydroxypropionsäure durch ein Verfahren nach Anspruch 1;
II) Dehydratisierung der 3-Hydroxypropionsäure unter Bildung von Acrylsäure.

3. Ein Verfahren zur Herstellung von Polyacrylaten, umfassend die Verfahrensschritte
I) Herstellung von 3-Hydroxypropionsäure durch ein Verfahren nach Anspruch 1,
II) Dehydratisierung der 3-Hydroxypropionsäure unter Bildung von Acrylsäure,
III) Radikalische Polymerisation der Acrylsäure.

4. Ein Verfahren zur Herstellung von Acrylsäureestern, umfassend die Verfahrensschritte
I) Herstellung von 3-Hydroxypropionsäure durch ein Verfahren nach Anspruch 1,
II) Dehydratisierung der 3-Hydroxypropionsäure unter Bildung von Acrylsäure,
III) Veresterung der Acrylsäure.

5. Verwendung einer gegenüber ihrem Wildtyp gentechnisch veränderten Bakterienzelle der Gattungen Corynebacterium oder Escherichia, welche die Eigenschaften a) und b) und c) aufweist:
a) eine im Vergleich zu ihrem Wildtyp gesteigerte Aktivität eines Enzyms E_{1b}, welches die Umsetzung von Phosphoenolpyruvat zu Oxalacetat katalysiert, wobei das Enzym E_{1b} eine Phosphoenolpyruvat-Carboxylase, welche von dem ppc-Gen kodiert wird, ist,
b) eine im Vergleich zu ihrem Wildtyp gesteigerte Aktivität eines Enzyms E₂, welches die Umsetzung von Aspartat zu Beta-Alanin katalysiert, und wobei das Enzym E₂ eine Aspartat-Decarboxylase, welche von dem panD-Gen kodiert wird, ist,
c) die gentechnisch veränderte Zelle ist durch eine Steigerung der Aktivität der Transportenzyme ausgewählt aus der Gruppe umfassend *multi-drug-resistance*-Proteine wie ebrA und ebrB, *multi-drug-efflux* Transporter wie blt- und bmr-Gene,
vom *E*. *coli* cycA-Gen kodierte Transportsysteme,
das hPAT1 Transportsystem,
das tGAT-B-, rGAT-B-, mGAT3-, mGAT2-,tGAT1 Transportsystem und
Das (B^{0,+}) Transportsystem aus Hase in der Lage, Beta-Alanin aus der Zelle herauszuschleusen,
zur Herstellung von 3-Hydroxypropionsäure.

## Claims

1. Process for preparing 3-hydroxypropionic acid, comprising the process steps of
i) the contacting of a bacterial cell which is genetically modified in relation to its wild type and is from the genera Corynebacterium or Escherichia, and which has the properties a) and b) and c):
a) an increased activity by comparison with its wild type of an enzyme E_{1b} which catalyses the conversion of phosphoenolpyruvate into oxaloacetate, the enzyme E_{1b} being a phosphoenolpyruvate carboxylase which is encoded by the ppc gene,
b) an increased activity by comparison with its wild type of an enzyme E₂ which catalyses the conversion of aspartate into beta-alanine, and where the enzyme E₂ is an aspartate decarboxylase which is encoded by the panD gene,
c) the genetically modified cell, through an increase in the activity of the transport enzymes selected from the group comprising *multi-drug resistance* proteins such as ebrA and ebrB,
*multi-drug efflux* transporters such as blt and bmr genes,
transport systems encoded by the *E*. *coli* cycA gene,
the hPAT1 transport system,
the tGAT-B, rGAT-B, mGAT3, mGAT2, tGAT1 transport system and
the hare (B^{0,+}) transport system,
able to export beta-alanine out of the cell,
with a nutrient medium containing carbohydrates or glycerol under conditions under which beta-alanine is formed from the carbohydrates or the glycerol and at least in part reaches the nutrient medium from the cell, so that a beta-alanine-containing nutrient medium is obtained,
ii) contacting the beta-alanine-containing nutrient medium with a further cell which is able to take up the beta-alanine and convert it into 3-hydroxy-propionic acid.

2. Method for producing acrylic acid, including the steps of the method
I) production of 3-hydroxypropionic acid by a method according to Claim 1;
II) dehydration of the 3-hydroxypropionic acid to form acrylic acid.

3. Method for producing polyacrylates, including the steps of the method
I) production of 3-hydroxypropionic acid by a method according to Claim 1;
II) dehydration of the 3-hydroxypropionic acid to form acrylic acid,
III) radical polymerization of the acrylic acid.

4. Method for producing acrylic esters, including the steps of the method
I) production of 3-hydroxypropionic acid by a method according to Claim 1,
II) dehydration of the 3-hydroxypropionic acid to form acrylic acid,
III) esterification of the acrylic acid.

5. Use of a genetically modified cell in relation to its wild type and is from the genera Corynebacterium or Escherichia, and which has the properties a) and b) and c):
a) an increased activity by comparison with its wild type of an enzyme E_{1b} which catalyses the conversion of phosphoenolpyruvate into oxaloacetate, the enzyme E_{1b} being a phosphoenolpyruvate carboxylase which is encoded by the ppc gene,
b) an increased activity by comparison with its wild type of an enzyme E₂ which catalyses the conversion of aspartate into beta-alanine, and where the enzyme E₂ is an aspartate decarboxylase which is encoded by the panD gene,
c) the genetically modified cell, through an increase in the activity of the transport enzymes selected from the group comprising
*multi-drug resistance* proteins such as ebrA and ebrB,
*multi-drug efflux* transporters such as blt and bmr genes,
transport systems encoded by the *E*. *coli* cycA gene,
the hPAT1 transport system,
the tGAT-B, rGAT-B, mGAT3, mGAT2, tGAT1 transport system and
the hare (B^{0,+}) transport system,
able to export beta-alanine out of the cell, for producing 3-hydroxypropionic acid.

## Revendications

1. Procédé pour la préparation d'acide 3-hydroxypropionique, comprenant les étapes de procédé :
i) la mise en contact d'une cellule de bactérie génétiquement modifiée par rapport à son type sauvage des genres Corynebacterium ou Escherichia, qui présente les propriétés a) et b) et c) :
a) une activité augmentée, par rapport à son type sauvage, d'une enzyme E_{1b}, qui catalyse la transformation de phosphoénolpyruvate en oxalacétate, l'enzyme E_{1b} étant une phosphoénolpyruvate-carboxylase, qui est codée par le gène ppc,
b) une activité augmentée, par rapport à son type sauvage, d'une enzyme E₂, qui catalyse la transformation d'aspartate en bêta-alanine, et l'enzyme E₂ étant une aspartate-décarboxylase, qui est codée par le gène panD,
c) la cellule est modifiée génétiquement par une augmentation de l'activité des enzymes de transport choisies dans le groupe comprenant les protéines multirésistantes aux médicaments, telles que ebrA et ebrB,
les transporteurs d'efflux de médicaments multiples, tels que les gènes blt et bmr,
les systèmes de transport codés par le gène cycA d'E. coli,
le système de transport hPAT1,
les systèmes de transport tGAT-B, rGAT-B, mGAT3, mGAT2, tGAT1 et
le système de transport (B^{0,+}) de lièvre en mesure d'excréter la bêta-alanine des cellules,
avec un milieu nutritif contenant des hydrates de carbone ou du glycérol, dans des conditions dans lesquelles on forme, à partir des hydrates de carbone ou du glycérol, la bêta-alanine, qui arrive au moins partiellement, des cellules, dans le milieu nutritif, de manière à obtenir un milieu nutritif contenant de la bêta-alanine ;
ii) la mise en contact du milieu nutritif contenant la bêta-alanine avec une autre cellule qui est en mesure d'absorber la bêta-alanine et de la transformer en acide 3-hydroxypropionique.

2. Procédé pour la préparation d'acide acrylique, comprenant les étapes de procédé :
I) préparation d'acide 3-hydroxypropionique par un procédé selon la revendication 1,
II) déshydratation de l'acide 3-hydroxypropionique avec formation d'acide acrylique.

3. Procédé pour la préparation de polyacrylates, comprenant les étapes de procédé :
I) préparation d'acide 3-hydroxypropionique par un procédé selon la revendication 1,
II) déshydratation de l'acide 3-hydroxypropionique avec formation d'acide acrylique,
III) polymérisation radicalaire de l'acide acrylique.

4. Procédé pour la préparation d'esters de l'acide acrylique, comprenant les étapes de procédé :
I) préparation d'acide 3-hydroxypropionique par un procédé selon la revendication 1,
II) déshydratation de l'acide 3-hydroxypropionique avec formation d'acide acrylique,
III) estérification de l'acide acrylique.

5. Utilisation d'une cellule de bactérie génétiquement modifiée par rapport à son type sauvage des genres Corynebacterium ou Escherichia, qui présente les propriétés a) et b) et c) :
a) une activité augmentée, par rapport à son type sauvage, d'une enzyme E_{1b}, qui catalyse la transformation de phosphoénolpyruvate en oxalacétate, l'enzyme E_{1b} étant une phosphoénolpyruvate-carboxylase, qui est codée par le gène ppc,
b) une activité augmentée, par rapport à son type sauvage, d'une enzyme E₂, qui catalyse la transformation d'aspartate en bêta-alanine, et l'enzyme E₂ étant une aspartate-décarboxylase, qui est codée par le gène panD,
c) la cellule est modifiée génétiquement par une augmentation de l'activité des enzymes de transport choisies dans le groupe comprenant les protéines de résistance multiple aux médicaments, telles que ebrA et ebrB,
les transporteurs d'efflux de médicaments multiples, tels que les gènes blt et bmr,
les systèmes de transport codés par le gène cycA d'E. coli,
le système de transport hPAT1,
les systèmes de transport tGAT-B, rGAT-B, mGAT3, mGAT2, tGAT1 et
le système de transport (B^{0,+}) de lièvre en mesure d'excréter la bêta-alanine des cellules,
pour la préparation d'acide 3-hydroxypropionique.
